# EUROPEAN PATENT SPECIFICATION

(11) **EP 0 570 575 B1**
(45) Date of publication and mention of the grant of the patent: **10.12.1997**
(21) Application number: 93901000.5
(22) Date of filing: 10.12.1992
(51) Int. Cl.: A61K 49/04, C07F 9/6561, C07F 9/6524

(54) **BICYCLOAZAMACROCYCLOPHOSPHONIC ACID, CONJUGATES, CONTRAST AGENTS AND PREPARATION**
Bicycloazamakrocyclophosphon-Säure, Komplexe, Konjugate, Kontrastmittel und Herstellung
ACIDES BICYCLOAZAMACROCYCLOPHOSPHONIQUES, CONJUGUES, AGENTS DE CONTRASTE ET LEUR PREPARATION

(30) Priority: 10.12.1991 US 805551
(43) Date of publication of application: 24.11.1993
(60) Divisional application: 96107948.0
(73) Proprietor: THE DOW CHEMICAL COMPANY, Midland, Michigan 48674 (US)
(72) Inventor: KIEFER, Garry, E., Lake Jackson, TX 77566 (US); SIMON, Jaime, Angleton, TX 77515 (US); GARLICH, Joseph, R., Lake Jackson, TX 77566 (US)
(74) Representative: Burford, Anthony Frederick
(86) International application number: US9210668
(87) International publication number: WO9311802

(56) References cited:
- EP-A- 0 352 218
- EP-A- 0 391 766
- EP-A- 0 430 863
- EP-A- 0 438 206
- WO-A-91/10645
- WO-A-91/10669

## Description

This invention concerns ligands that are bicyclopolyazamacrocyclophosphonic acids, and complexes and conjugates thereof, for use as contrast agents in magnetic resonance imaging (MRI). Some ligands and complexes are also useful as oral care agents and as scale inhibiting agents in water treatment systems. To better understand this invention, a brief background on MRI is provided in the following section.

### Background

MRI is a non-invasive diagnostic technique which produces well resolved cross-sectional images of soft tissue within an animal body, preferably a human body. This technique is based upon the property of certain atomic nuclei (e.g. water protons) which possess a magnetic moment [as defined by mathematical equations; see G. M. Barrow, Physical Chemistry, 3rd Ed., McGraw-Hill, NY (1973)] to align in an applied magnetic field. Once aligned, this equilibrium state can be perturbed by applying an external radio frequency (RF) pulse which causes the protons to be tilted out of alignment with the magnetic field. When the RF pulse is terminated, the nuclei return to their equilibrium state and the time required for this to occur is known as the relaxation time. The relaxation time consists of two parameters known as spin-lattice (T1) and spin-spin (T2) relaxation and it is these relaxation measurements which give information on the degree of molecular organization and interaction of protons with the surrounding environment.

Since the water content of living tissue is substantial and variations in content and environment exist among tissue types, diagnostic images of biological organisms are obtained which reflect proton density and relaxation times. The greater the differences in relaxation times (T1 and T2) of protons present in tissue being examined, the greater will be the contrast in the obtained image [*J. Magnetic Resonance* 33, 83-106 (1979)].

It is known that paramagnetic chelates possessing a symmetric electronic ground state can dramatically affect the T1 and T2 relaxation rates of juxtaposed water protons and that the effectiveness of the chelate in this regard is related, in part, to the number of unpaired electrons producing the magnetic moment [*Magnetic Resonance Annual*, 231 266, Raven Press, NY (1985)]. It has also been shown that when a paramagnetic chelate of this type is administered to a living animal, its effect on the T1 and T2 of various tissues can be directly observed in the magnetic resonance (MR) images with increased contrast being observed in the areas of chelate localization. It has therefore been proposed that stable, non-toxic paramagnetic chelates be administered to animals in order to increase the diagnostic information obtained by MRI [*Frontiers of Biol*. Energetics I, 752-759 (1978); *J. Nucl. Med*. 25, 506-513 (1984); Proc. of NMR Imaging Symp. (Oct. 26-27, 1980); F. A. Cotton et al., *Adv. Inorg. Chem*. 634-639 (1966)]. Paramagnetic metal chelates used in this manner are referred to as contrast enhancement agents or contrast agents.

There are a number of paramagnetic metal ions which can be considered when undertaking the design of an MRI contrast agent. In practice, however, the most useful paramagnetic metal ions are gadolinium (Gd⁺³), iron (Fe⁺³), manganese (Mn⁺²) and (Mn⁺³), and chromium (Cr⁺³), because these ions exert the greatest effect on water protons by virtue of their large magnetic moments. In a non-complexed form (e.g. GdCl₃), these metal ions are toxic to an animal, thereby precluding their use in the simple salt form. Therefore, a fundamental role of the organic cheating agent (also referred to as a ligand) is to render the paramagnetic metal non-toxic to the animal while preserving its desirable influence on T1 and T2 relaxation rates of the surrounding water protons.

Art in the MRI field is quite extensive, such that the following summary, not intended to be exhaustive, is provided only as a review of this area and other compounds that are possibly similar in structure. US-A-4,899,755 discloses a method of alternating the proton NMR relaxation times in the liver or bile duct of an animal using Fe⁺³-ethylene-bis(2-hydroxyphenylglycine) complexes and its derivatives, and suggests among various other compounds the possible use of a pyridine macrocyclomethylenecarboxylic acid. US-A-4,880,008 (a CIP of US-A-4,899,755) discloses additional imaging data for liver tissue of rats, but without any additional complexes being shown. US-A-4,980,148 disclose gadolinium complexes for MRI which are non-cyclic compounds. C. J. Broan et al., *J. Chem. Soc., Chem. Commun*., 1739-1741 (1990) describe some bifunctional macrocyclic phosphinic acid compounds. C. J. Broan et al., *J. Chem. Soc., Chem. Commun*., 1738-1739 (1990) describe compounds that are triazabicyclo compounds. I. K. Adzamli et al., *J. Med. Chem.* 32, 139-144 (1989) describes acyclic phosphonate derivatives of gadolinium complexes for NMR imaging.

At the present time, the only commercial contrast agent available in the U.S.A. is the complex of gadolinium with diethylenetriaminepentaacetic acid (DTPA-Gd⁺³-MAGNEVIST™ by Schering AG). MAGNEVIST™ is considered as a non-specific/perfusion agent since it freely distributes in extracellular fluid followed by efficient elimination through the renal system. MAGNEVIST™ has proven to be extremely valuable in the diagnosis of brain lesions since the accompanying breakdown of the blood/brain barrier allows perfusion of the contrast agent into the affected regions. In addition to MAGNEVIST™, Guerbet is commercially marketing a macrocyclic perfusion agent (DOTAREM™) which presently is only available in Europe. A number of other potential contrast agents are in various stages of development.

Surprisingly, it has now been found that various bicyclopolyazamacrocyclophosphonic acid ligands can be contrast agents. Furthermore, these ligands may have their charge modified, i.e. by the structure of the ligand and metal selected, which can effect their ability to be more site specific. Specifically, the present invention is directed to novel ligands that are bicyclopolyazamacrocyclophosphonic acid compounds of the formula 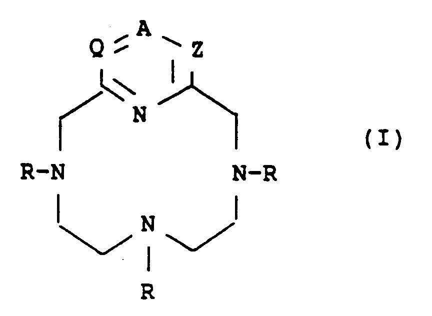 wherein: 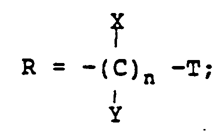 where:
X and Y are independently H, OH, C₁-C₃ alkyl or COOH;
n is an integer of 1, 2 or 3; with the proviso that: when n is 2, then the sum of X and Y must equal two or more H; and when n is 3, then the sum of X and Y must equal three or more H;
T is H, C₁-C₁₈ alkyl, COOH, OH, SO₃H, 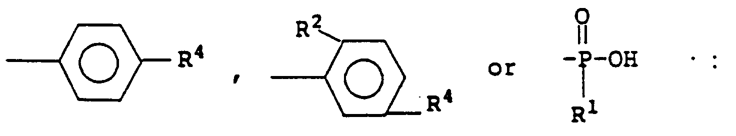
where: R¹ is -O-(C₁-C₅ alkyl);
R⁴ is H, NO₂, NH₂, isothiocyanato, semicarbazido, thiosemicarbazido, maleimido, bromoacetamido or carboxyl;
R² is H or OH; with the proviso that when R² is OH, then the R term containing the R² must have all X and Y equal to H;
with the proviso that at least one T must be P(O)R¹OH, and with the proviso that when one T is 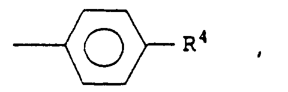
then one X or Y of that R term may be COOH and all other X and Y terms of that R term must be H;
A is CH, N, C-Br, C-Cl, C-OR³, C-OR⁸, N⁺-R⁵X⁻, 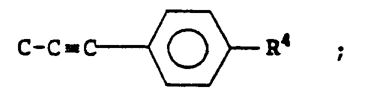
R³ is H, C₁-C₅ alkyl, benzyl, or benzyl substituted with at least one R⁴;
R⁴ is defined as above;
R⁵ is C₁-C₁₆ alkyl, benzyl, or benzyl substituted with at least one R⁴;
R⁸ is C₁-C₁₆ alkylamino;
X⁻ is Cl⁻, Br⁻, I⁻ or H₃CCO₂⁻;
Q and Z independently are CH, N, N⁺-R⁵X⁻, C-CH₂-OR³ or C-C(O)-R⁶;
R⁵ is defined as above;
R⁶ is -O-(C₁-C₃ alkyl), OH or NHR⁷;
R⁷ is C₁-C₅ alkyl or a biologically active material;
X⁻ is defined as above; or
pharmaceutically-acceptable salts thereof;
with the proviso that:
a) when Q, A or Z is N or N⁺-R⁵X⁻, then the other two groups must be CH;
b) when A is C-Br, C-Cl, C-OR³ or C-OR⁸, then both Q and Z must be CH;
c) the sum of the R⁴, R⁷ and R⁸ terms, when present, may not exceed one; and
d) only one of Q or Z can be C-C(O)-R⁶ and when one of Q or Z is C-C(O)-R⁶, then A must be CH.

When in the R term three T equal P(O)R¹OH, then the ligands are useful as contrast agents.

Particularly preferred are those ligands of Formula (I) where:
X and Y are H;
n is 1; or
A, Q and Z are CH.

Bifunctional ligands of Formula (I) are desirable to prepare the conjugates of this invention. Such ligands must have:
one R term where the T moiety is 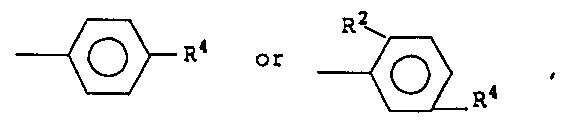 where R² and R⁴ are defined as above, especially where (a) in the two R terms not containing an R⁴ term, both T terms are P(O)R¹OH, where R¹ is defined as above or (b) where in the two R terms not containing an R⁴ term, one T term is a COOH and the other T term is P(O)R¹OH, where R¹ is defined as above; preferably that moiety of the above T term containing R⁴ where one of X or Y of that term is COOH; and also preferred are those ligands where n is 1 and/or the remaining X and Y terms are H; or
A is C-OR³ or C-OR⁸, where R³ and R⁸ are defined as above or 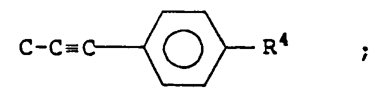 where R⁴ is defined as above; or
A is CH, and one of Q or Z is CH and the other is C-C(O)-R⁶, where R⁶ is defined as above;
   especially those ligands where R⁶ is NHR⁷, where R⁷ is a biologically active material.

The ligands of Formula (I) may be complexed with various metal ions, such as gadolinium (Gd⁺³), iron (Fe⁺³), and manganese (Mn⁺²), with Gd⁺³ being preferred. The complexes so formed can be used by themselves or can be attached, by being covalently bonded to a larger molecule such as a dextran, a polypeptide or a biologically active molecule, including an antibody or fragment thereof, and used tor diagnostic purposes. Such conjugates and complexes are useful as contrast agents.

The complexes and conjugates of this invention can be designed to provide a specific overall charge which advantageously influences the *in vivo* biolocalization and image contrast. For example, when the metal ion is + 3 the following can be obtained:
an overall neutral charge - when
in the three R terms T is P(O)R¹OH, where R¹ is -O-(C₁-C₅ alkyl), and n is 1; or
in the three R terms T is P(O)R¹OH, where R¹ is C₁-C₅ alkyl, and n is 1; or
an overall charge of + 1 - when
one of A, Q or Z is N⁺-R⁵X⁻, where R⁵ and X⁻ are defined as above; and in one R term, the T moiety is P(O)R¹OH, ; and in the other two R terms, the T moiety is COOH or P(O)R¹OH, ; and all X and Y terms are H.

Both the complexes and conjugates may be formulated to be in a pharmaceutically acceptable form for administration to an animal.

Use of the ligands of Formula (I) with other metal ions for diagnosis of disease states such as cancer is possible. The use of those complexes and conjugates is discussed in another copending application.

The compounds of Formula (I) are numbered for nomenclature purposes as follows:

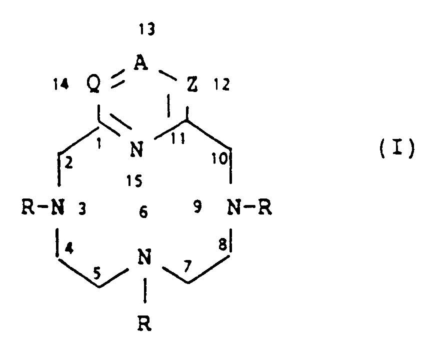

One aspect of the present invention concerns development of contrast agents having synthetic modifications to the paramagnetic chelate enabling site specific delivery of the contrast agent to a desired tissue. The advantage being increased contrast in the areas of interest based upon tissue affinity as opposed to contrast arising from non-specific perfusion which may or may not be apparent with an extracellular agent. The specificity of the ligand of Formula (I) may be controlled by adjusting the total charge and lipophilic character of the complex. when the overall charge of the complex is 0 (thus neutral), the complex may have the ability to cross the blood brain barrier and normal brain uptake may be possible.

Tissue specificity may also be realized by ionic or covalent attachment of the chelate to a naturally occurring or synthetic molecule having specificity for a desired target tissue. One possible application of this approach is through the use of chelate conjugated monoclonal antibodies which would transport the paramagnetic chelate to diseased tissue enabling visualization by MRI. In addition, attachment of a paramagnetic chelate to a macromolecule can further increase the contrast agent efficiency resulting in improved contrast relative to the unbound chelate. Recent work by Lauffer (US-A-4,880,008 and US-A-4,899,755) has demonstrated that variations in lipophilicity can result in tissue-specific agents and that increased lipophilic character favors non-covalent interactions with blood proteins resulting in enhancement of relaxivity.

Additionally, the present contrast agents of Formula (I) which are neutral in charge are particularly preferred for forming the conjugates of this invention since undesirable ionic interactions between the chelate and protein are minimized which preserves the antibody immunoreactivity. Also the present neutral complexes reduce the osmolarity relative to DTPA-Gd⁺³, which may alleviate the discomfort of injection.

While not wishing to be bound by theory, it is believed that when a charged complex is made (e.g. possibly -2 or -3 for bone, -1 for liver, or + 1 for heart), the variations in that chelate ionic charge can influence biolocalization. Thus, if the antibody or other directing moiety is also specific for the same site, then the conjugate displays two portions to aid in site specific delivery.

The terms used in Formula (I) are further defined as follows. "C₁-C₃ alkyl", "C₁-C₅ alkyl", "C₁-C₁₈ alkyl", include both straight and branched chain alkyl groups. An "animal" includes a warmblooded mammal, preferably a human being.

"Biologically active material" refers to a dextran, peptide, or molecules that have specific affinity for a receptor, or preferably antibodies or antibody fragments.

"Antibody" refers to any polyclonal, monoclonal, chimeric antibody or heteroantibody, preferably a monoclonal antibody; "antibody fragment" includes Fab fragments and F(ab')₂ fragments, and any portion of an antibody having specificity toward a desired epitope or epitopes. When using the term "radioactive metal chelate/antibody conjugate" or "conjugate", the "antibody" is meant to include whole antibodies and/or antibody fragments, including semisynthetic or genetically engineered variants thereof. Possible antibodies are 1116-NS-19-9 (anti-colorectal carcinoma), 1116-NS-3d (anti-CEA), 703D4 (anti-human lung cancer), 704A1 (anti-human lung cancer), CC49 (anti-TAG-72), CC83 (anti-TAG-72) and 872.3. The hybridoma cell lines 1116-NS-19-9, 1116-NS-3d, 703D4, 704A1, CC49, CC83 and B72.3 are deposited with the American Type Culture Collection, having the accession numbers ATCC HB 8059, ATCC CRL 8019, ATCC HB 8301, ATCC HB 8302, ATCC HB 9459, ATCC HB 9453 and ATCC HB 8108, respectively.

As used herein, "complex" refers to a complex of the compound of Formula (I) complexed with a metal ion, where at least one metal atom is chelated or sequestered; "conjugate" refers to a metal ion chelate that is covalently attached to a biologically active agent, especially an antibody or antibody fragment. The terms "bifunctional coordinator", "bifunctional cheating agent" and "functionalized chelant" are used interchangeably and refer to compounds that have a chelant moiety capable of cheating a metal ion and a moiety covalently bonded to the chelant moiety that is capable of serving as a means to covalently attach to an antibody or antibody fragment.

The bifunctional cheating agents described herein (represented by Formula I) can be used to chelate or sequester the metal ions so as to form metal ion chelates (also referred to herein as "complexes"). The complexes, because of the presence of the functionalizing moiety (represented by R⁴ or R⁸ in Formula I), can be covalently attached to biologically active materials, such as dextran, molecules that have specific affinity for a receptor, or preferably covalently attached to antibodies or antibody fragments. Thus the complexes described herein may be covalently attached to an antibody or antibody fragment or have specific affinity for a receptor and are referred to herein as "conjugates".

As used herein, "pharmaceutically-acceptable salts" means any salt or mixtures of salts of a compound of formula (I) which is sufficiently non-toxic to be useful in therapy or diagnosis of animals, preferably mammals. Thus, the salts are useful in accordance with this invention. Representative of those salts formed by standard reactions from both organic and inorganic sources include, for example, sulfuric, hydrochloric, phosphoric acetic, succinic, citric, lactic, maleic, fumaric, palmitic, cholic, palmoic, mucic, glutamic, gluconic acid, d-camphoric, glutaric₁ glycolic, phthalic, tartaric, formic, lauric, steric, salicylic, methanesulfonic, benzenesulfonic, sorbic, picric, benzoic, cinnamic acids and other suitable acids. Also included are salts formed by standard reactions from both organic and inorganic sources such as ammonium or 1-deoxy-1-(methylamino)-D-glucitol, alkali metal ions, alkaline earth metal ions, and other similar ions. Particularly preferred are the salts of the compounds of formula (I) where the salt is potassium, sodium, ammonium. Also included are mixtures of the above salts.

### Detailed Description of the Process

The compounds of Formula (I) are prepared by various processes. Typical general synthetic approaches to such processes are provided by the reaction schemes given below.

In Scheme 1, compounds are prepared wherein X and Y = H, n = 1 (but would also apply if n = 2 or 3 with the corresponding change in the reagent), T = PO₃H₂, and Q, A and Z = CH.

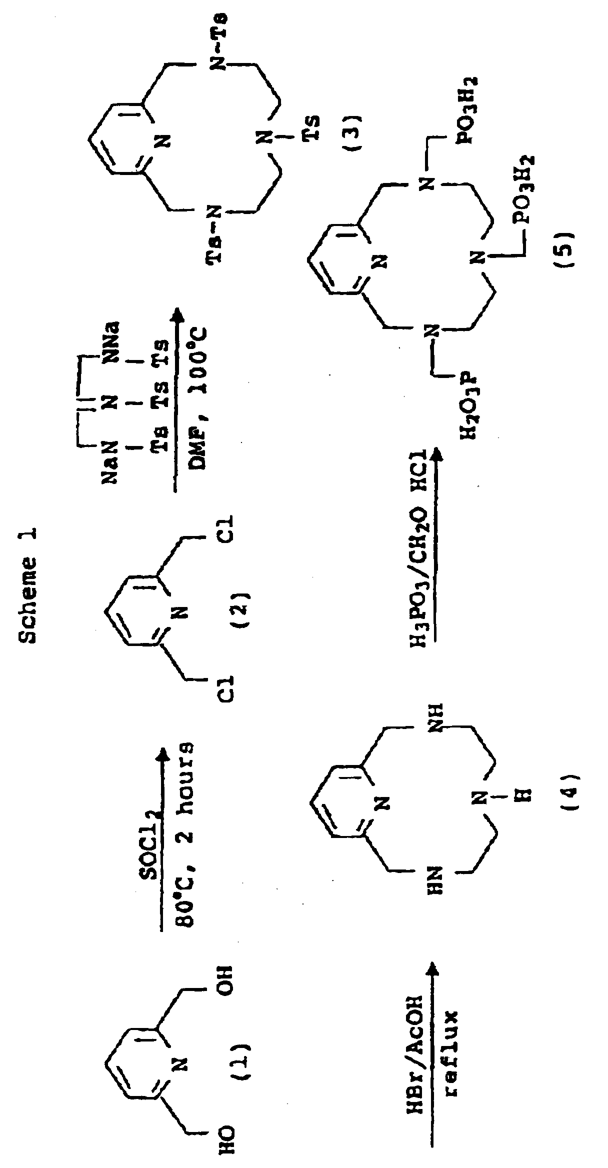

Scheme 2 prepares the compounds of Formula (I) wherein X and Y = H, n = 1 (but would also apply if n = 2 or 3 with the corresponding change in the reagent), T = 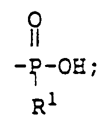 where R¹ = -O-(C₁-C₅ alkyl); and Q, A and Z = CH.

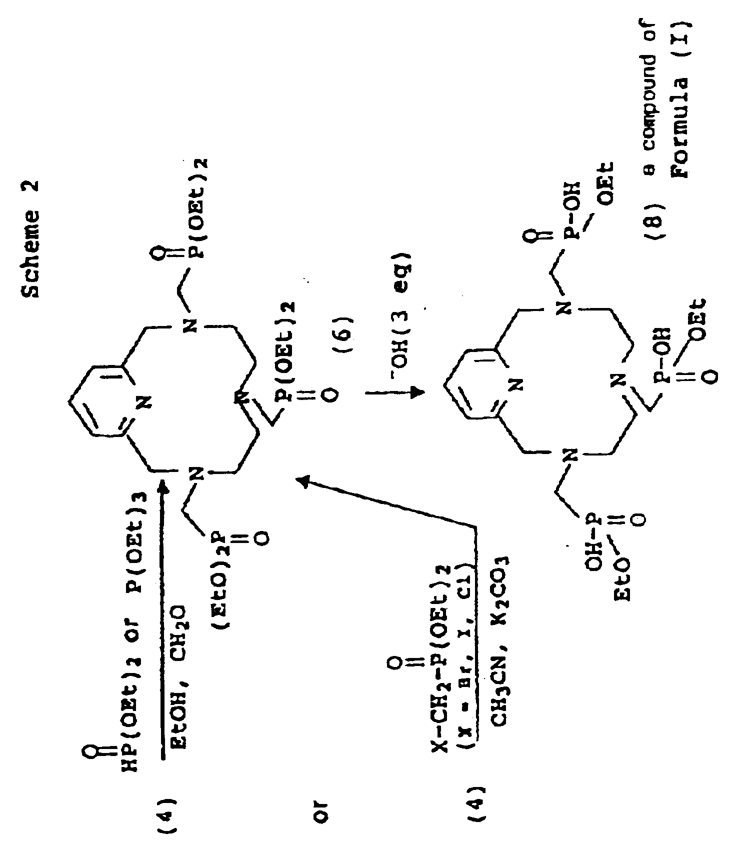

Scheme 3 prepares the compounds of Formula (I) wherein X and Y = H, n = 1 (but would also apply if n = 2 or 3 with the corresponding change in the reagent), T = 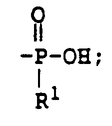 where R¹ = -O-(C₁-C₅ alkyl); A = C-Br, and Q and Z = CH.

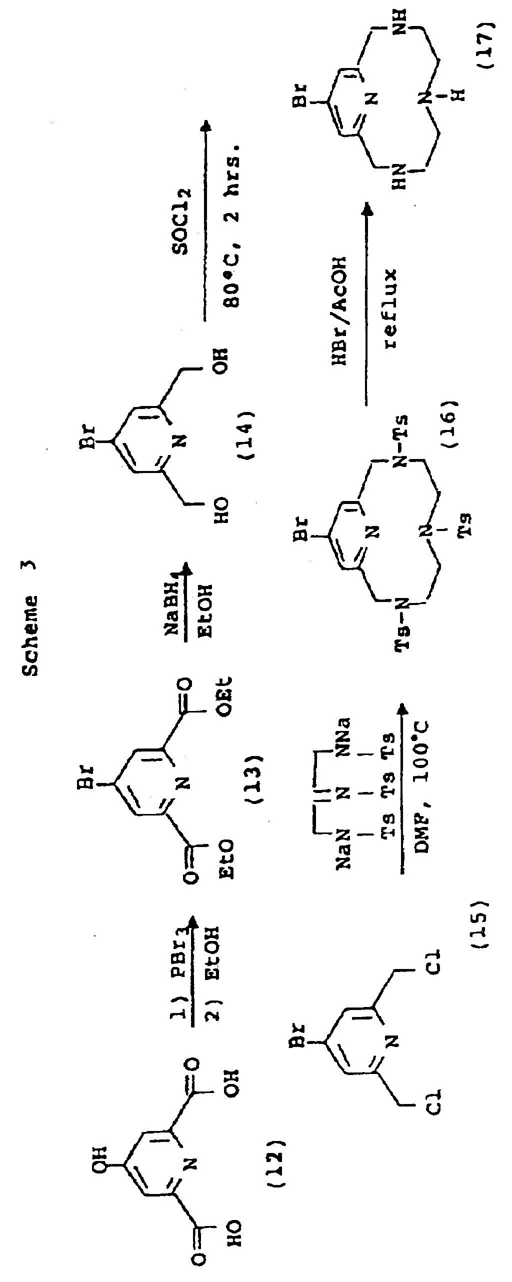

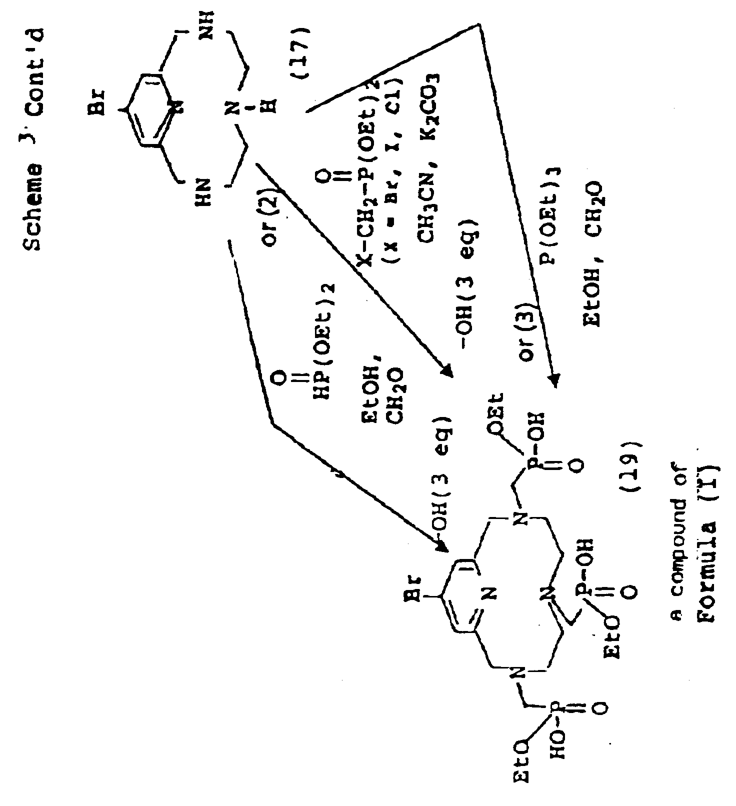

Scheme 4 prepares the compounds of Formula (I) wherein X and Y = H, n = 1 (but would also apply if n = 2 or 3 with the corresponding change in the reagent), T = 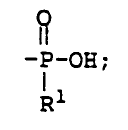 where R¹ = -O-(C₁-C₅ alkyl); A = 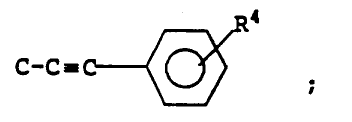 R⁴ = H, NO₂, NH₂ or SCN; and Q and Z = CH.

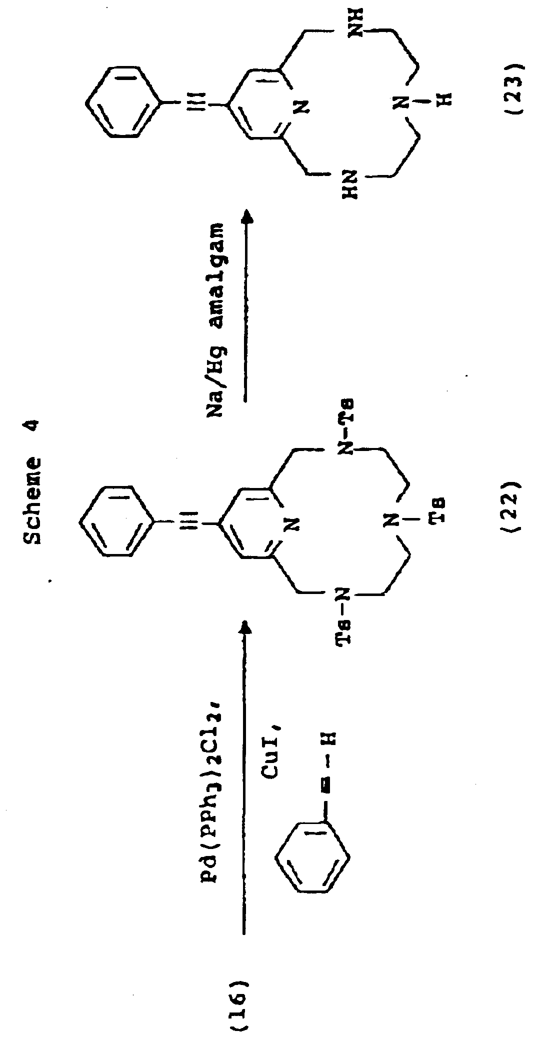

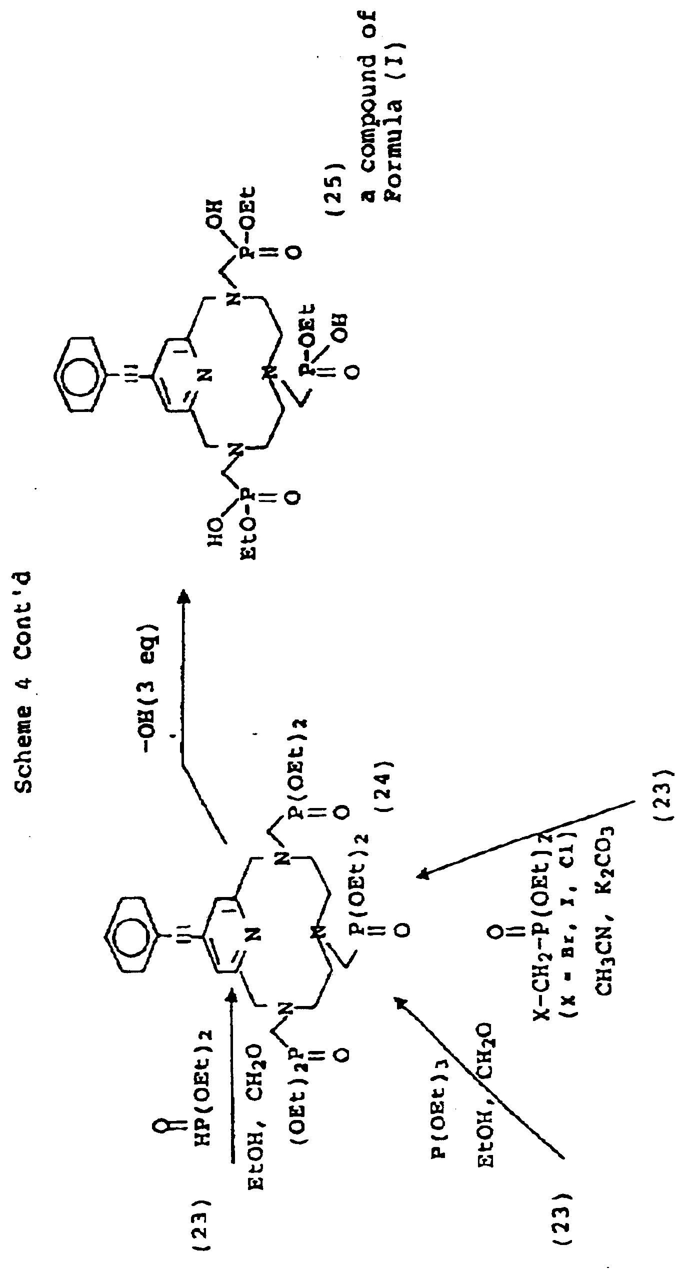

Scheme 5 prepares the compounds of Formula (I) wherein X and Y = H, n = 1 (but would also apply if n = 2 or 3 with the corresponding change in the reagent), T = 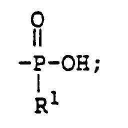 where R¹ = -O-(C₁-C₅ alkyl)
A = C-OR⁸, where R⁸ = C₁-C₅ alkylamino; and
Q and Z = CH.

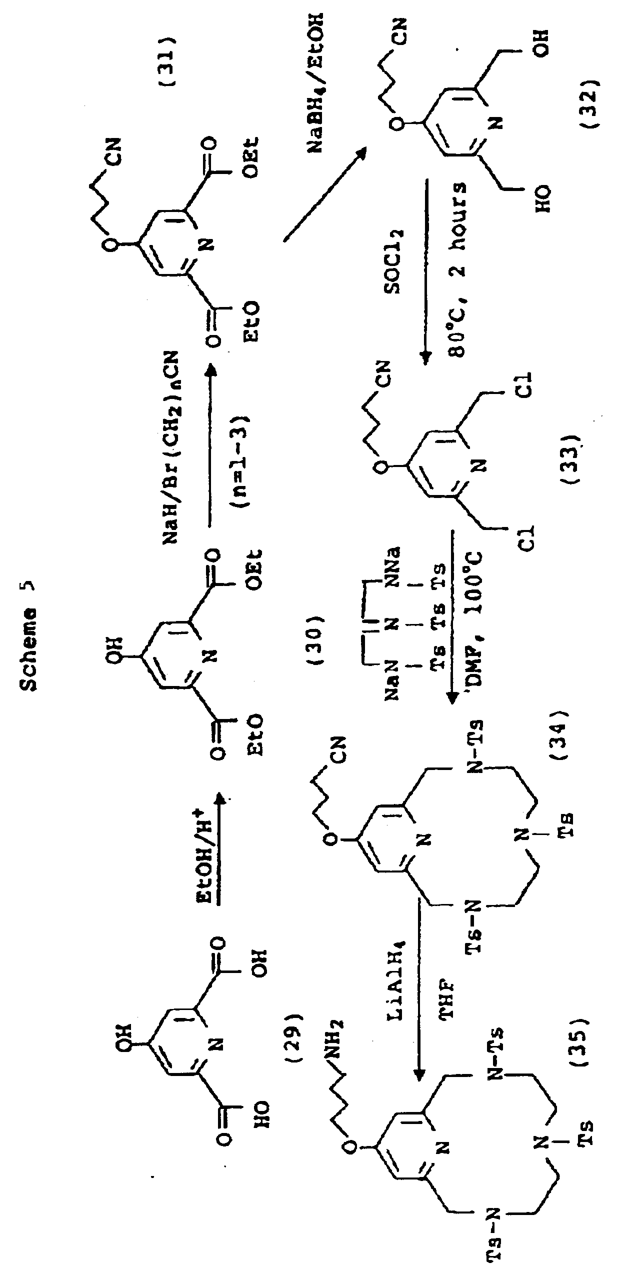

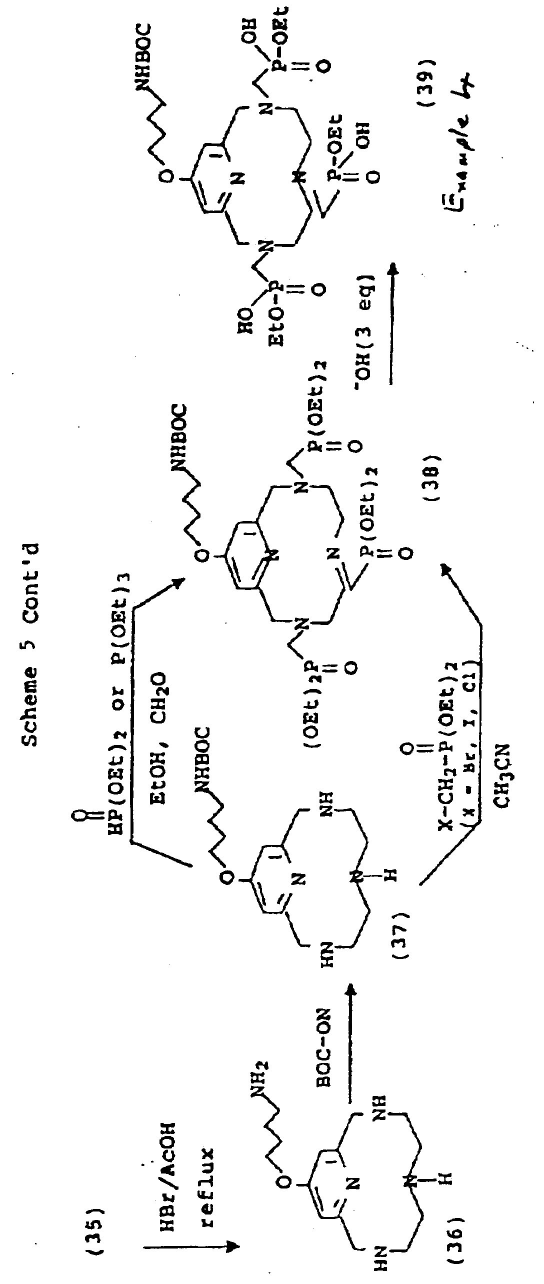

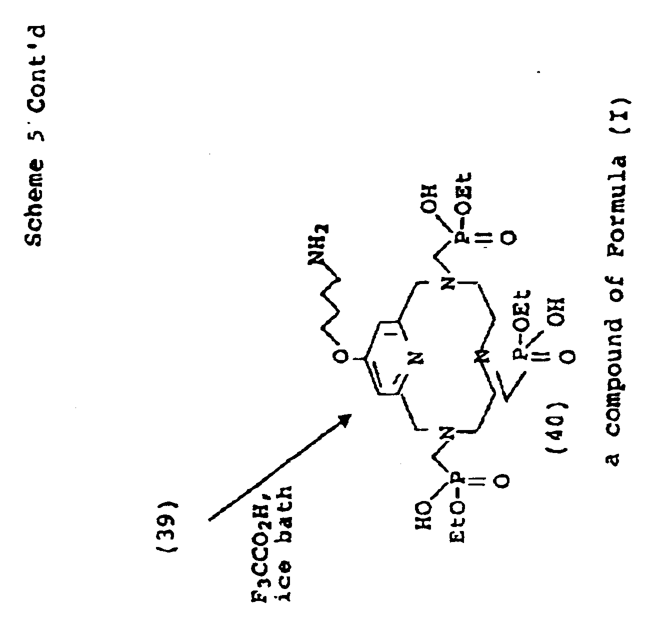

Scheme 6 prepares the compounds of Formula (I) wherein X and Y = H, n = 1 (but would also apply if n = 2 or 3 with the corresponding change in the reagent), T = 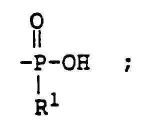 where R¹ = -O-(C₁-C₅ alkyl);
Z = C-C(O)-R⁶, where R⁶ = OH; and Q and A = CH.

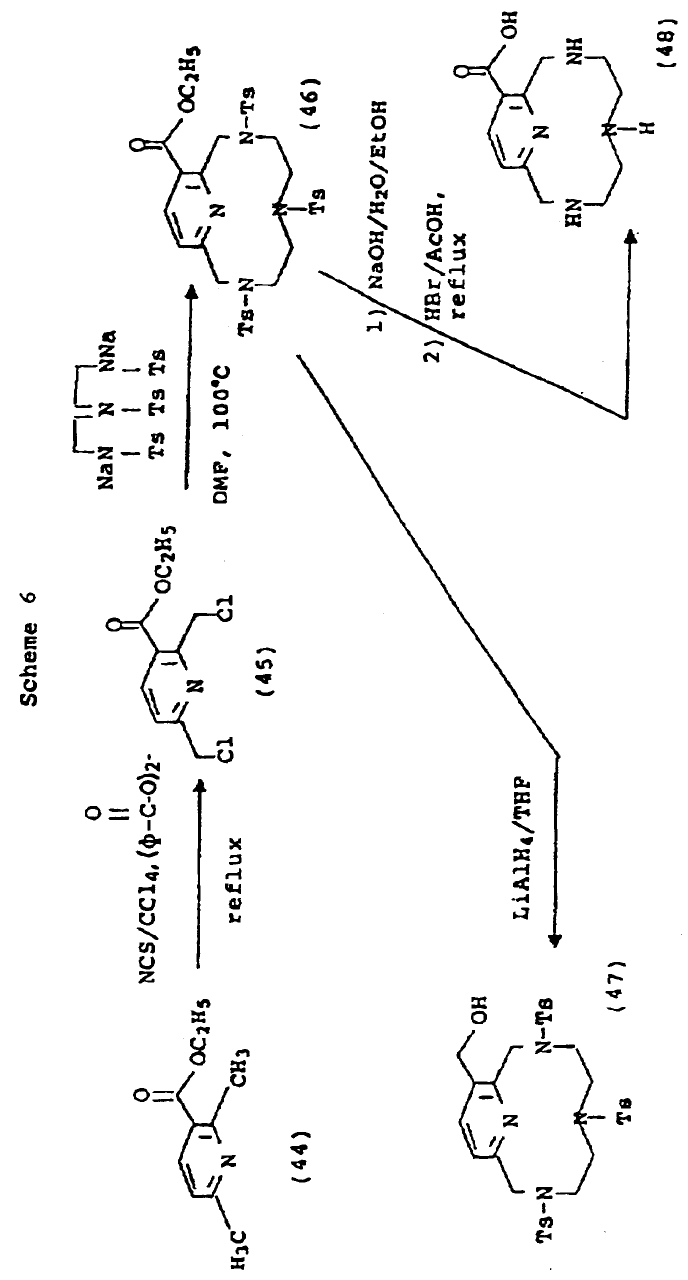

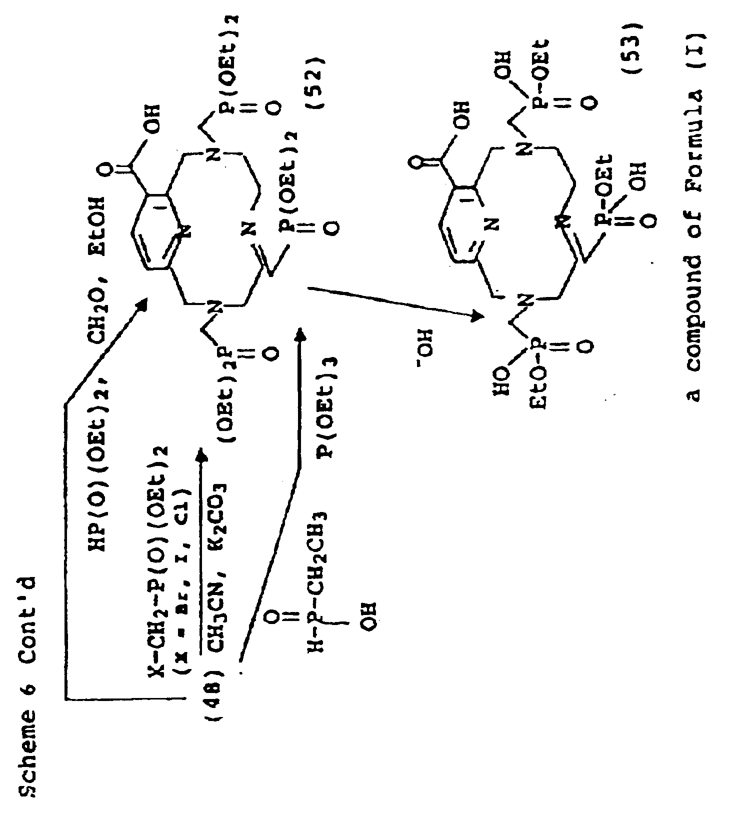

Scheme 7 prepares the compounds of Formula (I) wherein X and Y = H, n = 1 (but would also apply if n = 2 or 3 with the corresponding change in the reagent), T = 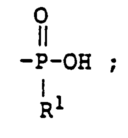 where R¹ = -O-(C₁-C₅ alkyl);
Z = C-CH₂-OR³ where R³ = benzyl; and
Q and A = CH.

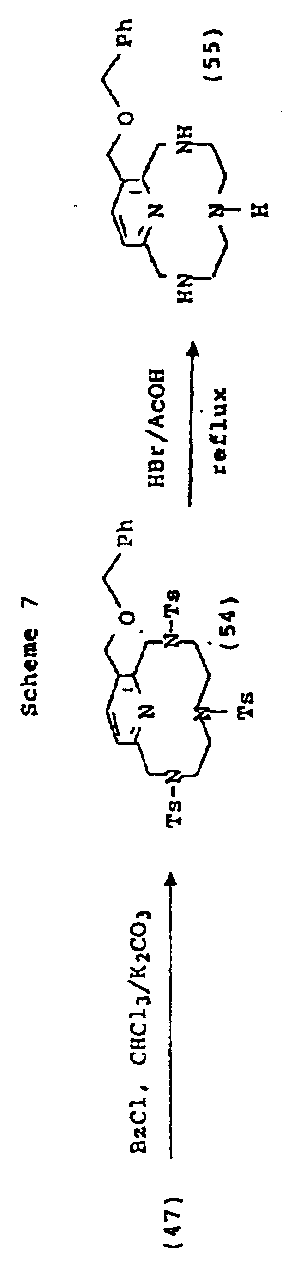

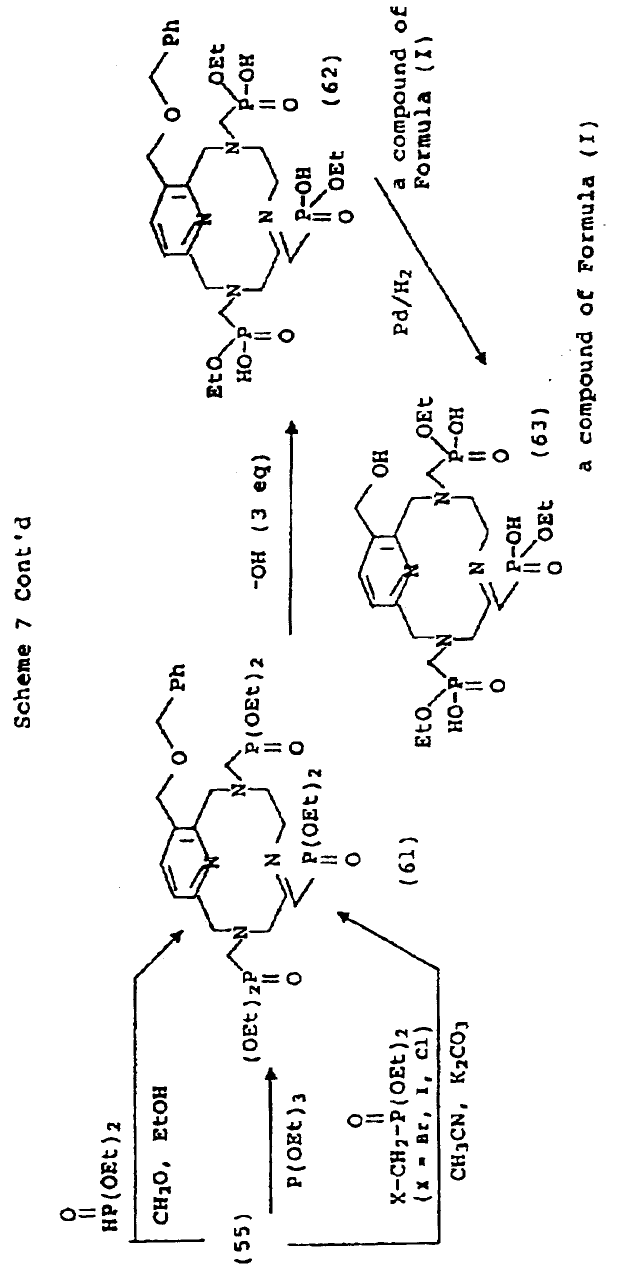

Scheme 8 prepares the compounds of Formula (I) wherein X and Y = H, n = 1 (but would also apply if n = 2 or 3 with the corresponding change in the reagent), T = 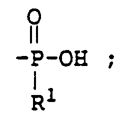 where R¹ = -O-(C₁-C₅ alkyl)
A = N or N-R⁵; R⁵ = C₁-C₁₆ alkyl halide; and
Q and Z = CH.

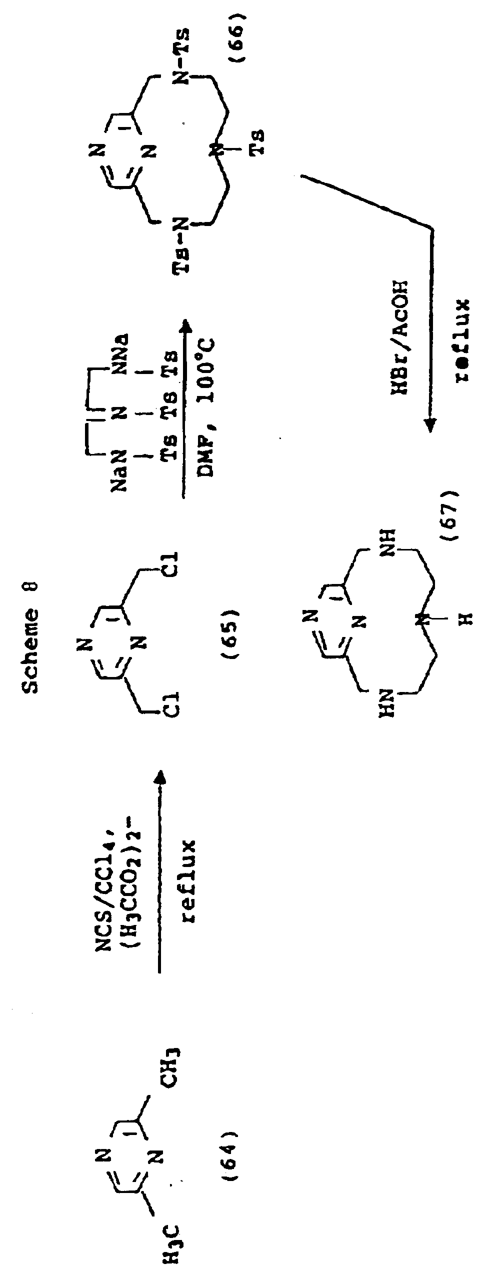

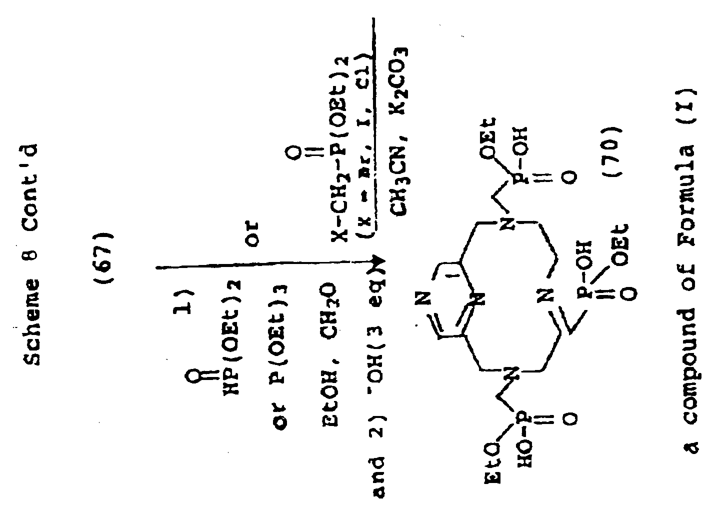

Scheme 9 prepares the compounds of Formula (I) wherein X and Y = H, n = 1 (but would also apply if n = 2 or 3 with the corresponding change in the reagent), T = 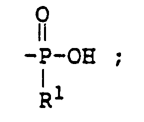 where R¹ = -O-[C₁-C₅ alkyl);
Q = N-R⁵; R⁵ = C₁-C₁₆ alkyl halide; and
A and Z = CH.

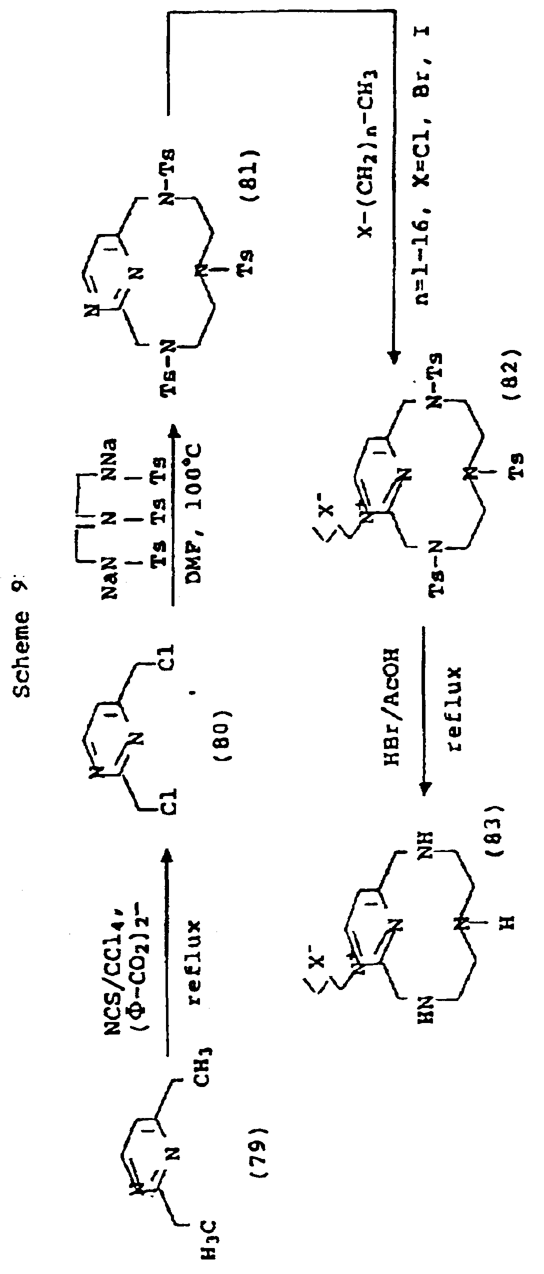

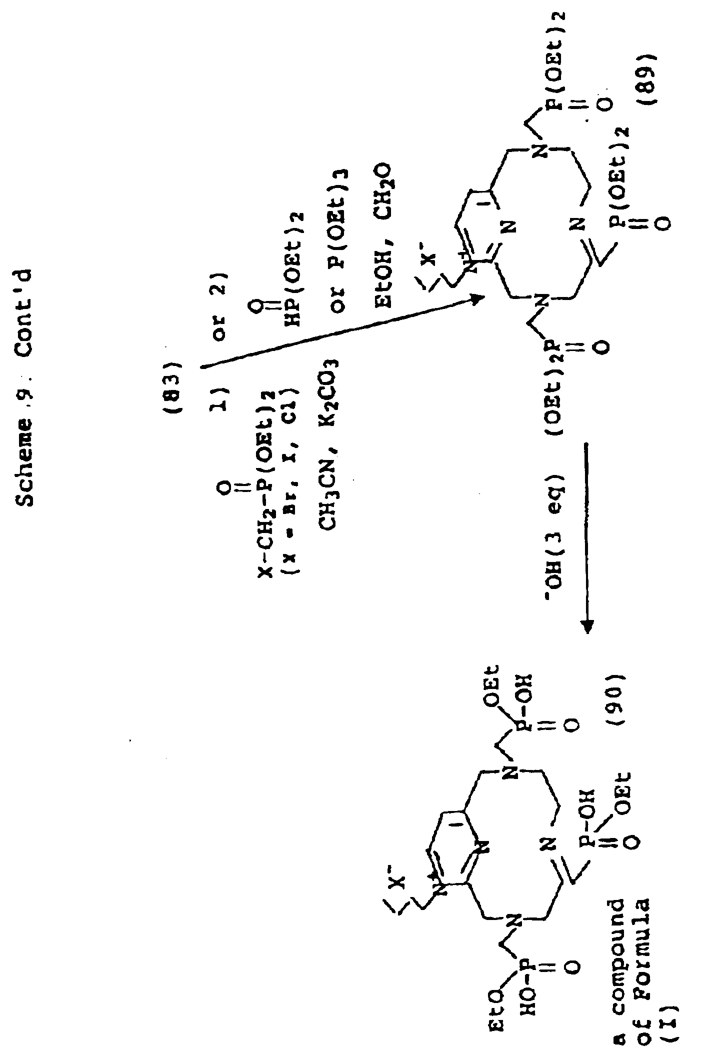

Scheme 10 prepares the compounds of Formula (I) wherein X and Y = H, n = 1 (but would also apply if n = 2 or 3 with the corresponding change in the reagent), T = 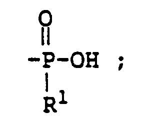 where R¹ = -O-(C₁-C₅ alkyl)
Q = N or N-R⁵, R⁵ = C₁-C₁₆ alkyl halide; and
A and Z = CH.

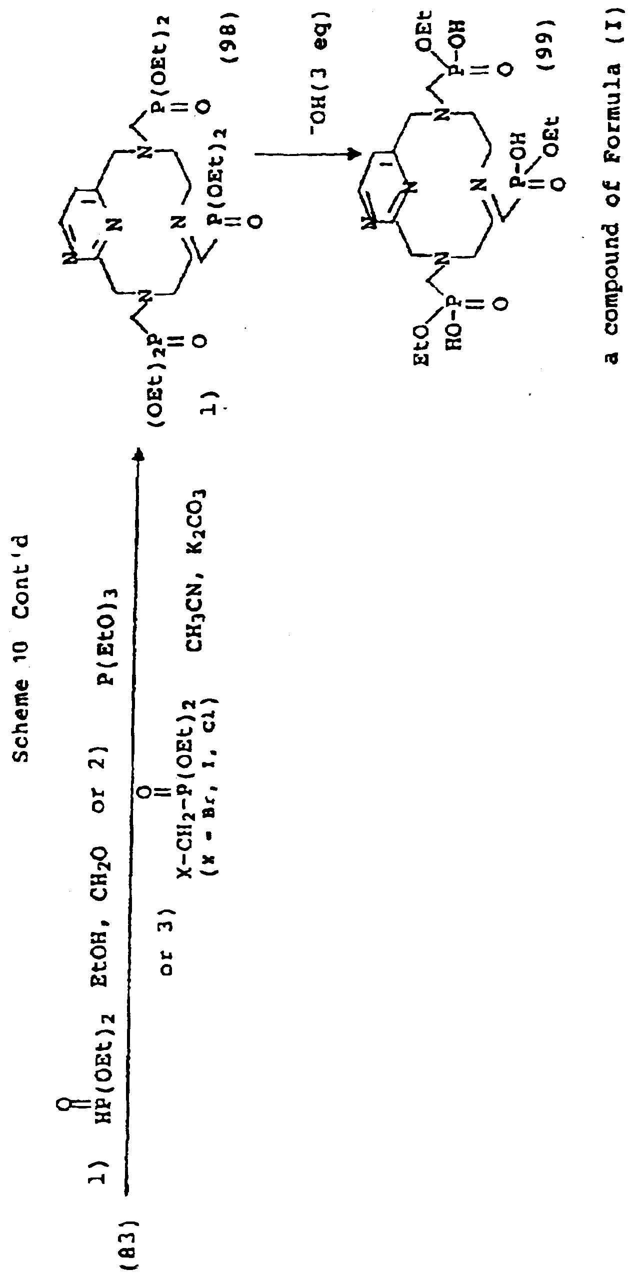

Scheme 11 prepares the compounds of Formula (I) wherein X and Y = H, n = 1 (but would also apply if n = 2 or 3 with the corresponding change in the reagent), R at the 3 position has T = 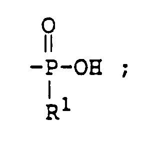 where R¹ = or -O-(C₁-C₅ alkyl); and the other two R terms have T = COOH; and A, Q and Z = CH.

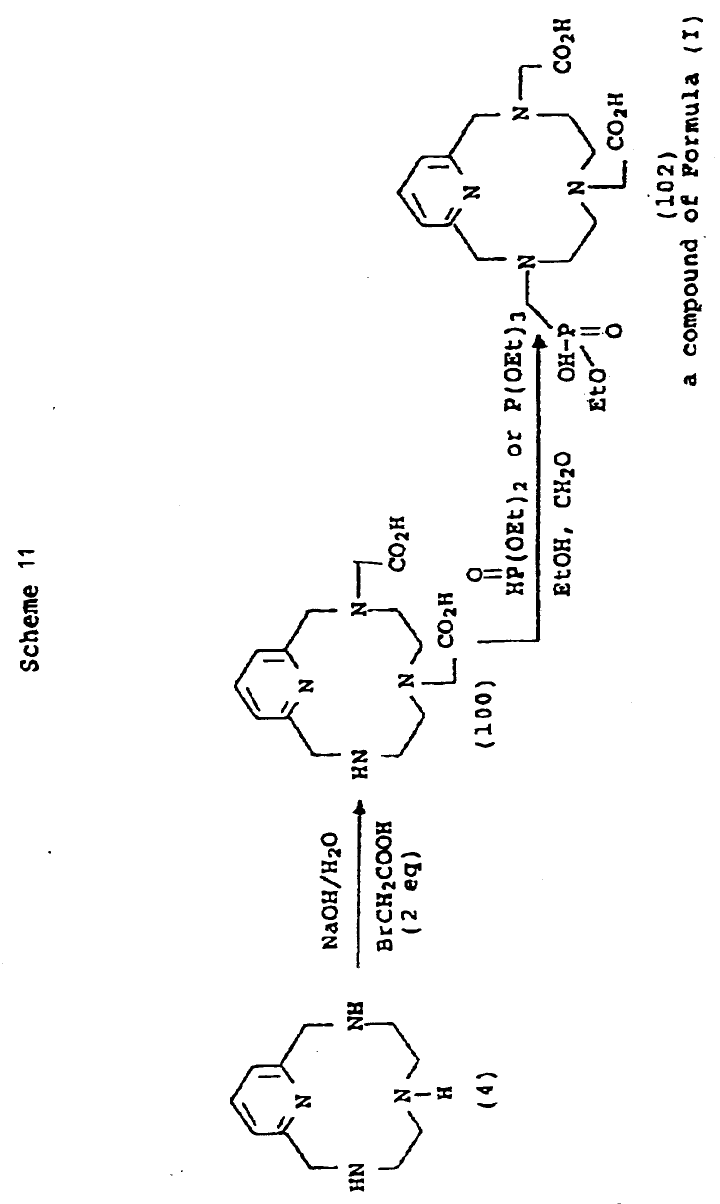

Scheme 12 prepares the compounds of Formula (I) wherein X and Y = H, n = 1 (but would also apply if n = 2 or 3 with the corresponding change in the reagent), R at the 3 and 6 positions have T = 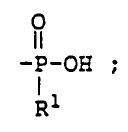 where R¹ = or -O-(C₁-C₅ alkyl); and the other R terms at the 9 position has T= COOH; and A, Q and Z = CH.

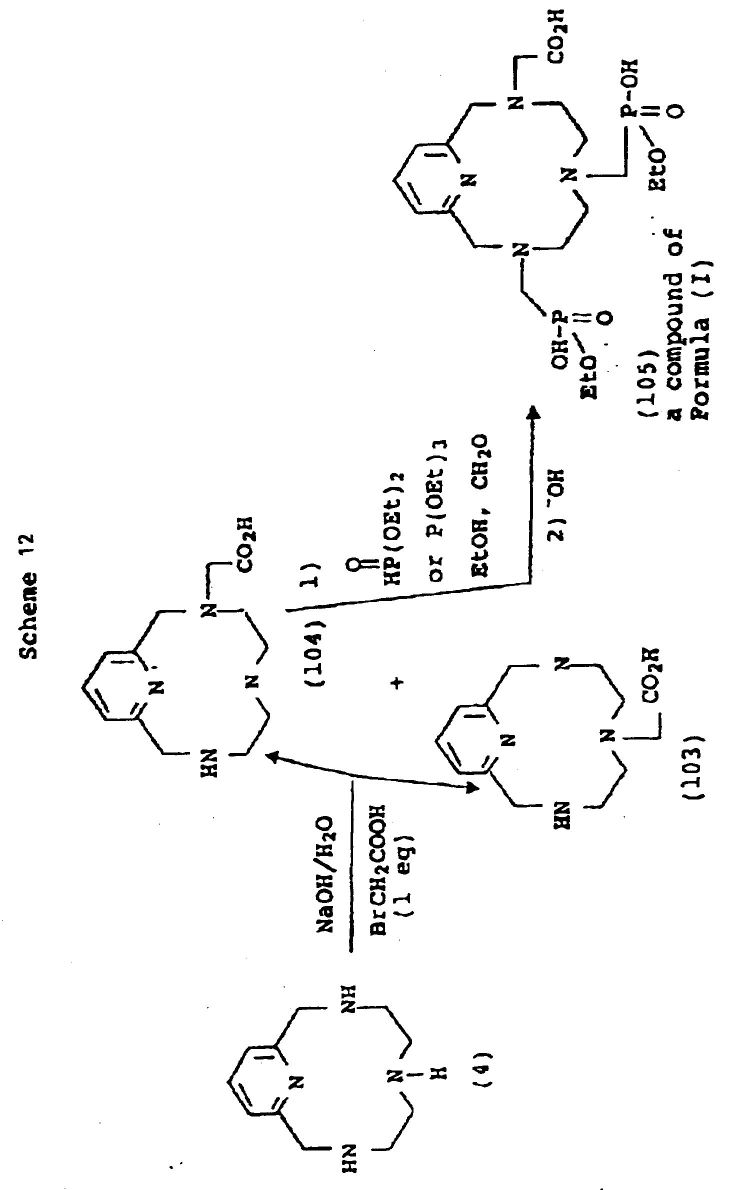

Scheme 13 prepares the compounds of Formula (I) wherein X and Y = H, n = 1 (but would also apply if n = 2 or 3 with the corresponding change in the reagent), R terms at the 3 and 9 positions have T = 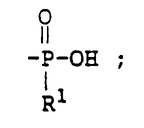 where R¹ = or O-(C₁-C₅ alkyl); and the other R term at the 6 position has T = COOH; and A, Q and Z = CH.

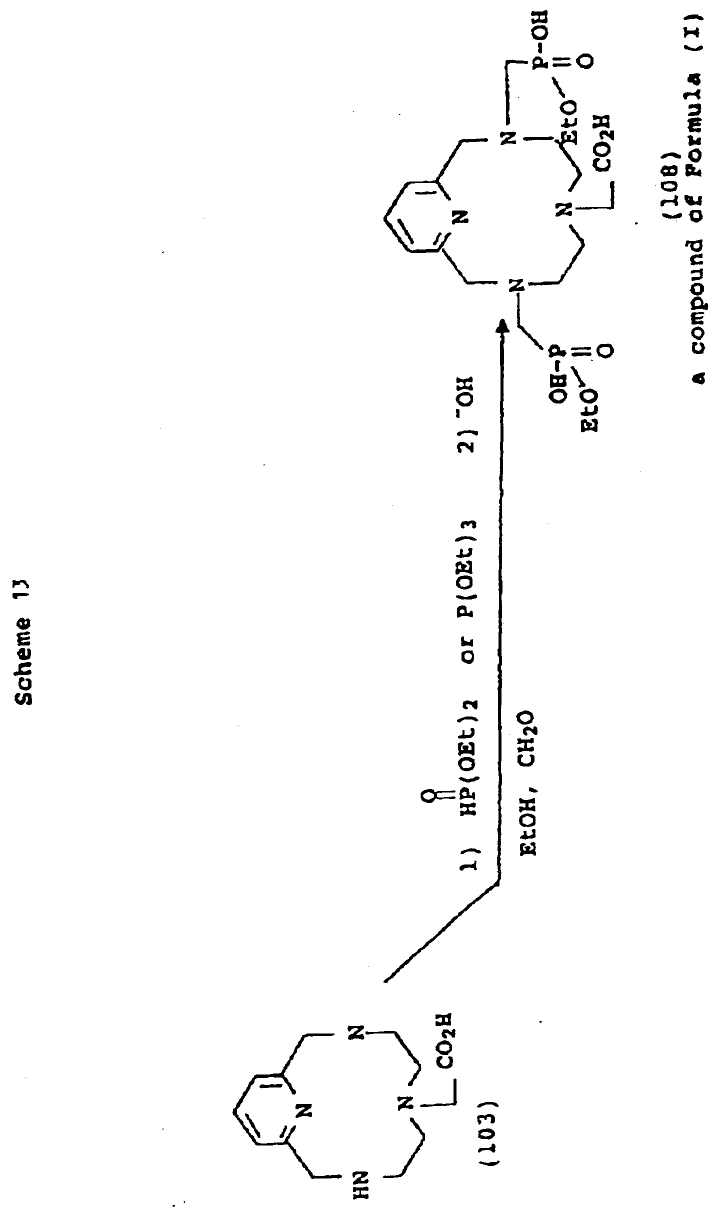

Scheme 14 prepares the compounds of Formula (I) wherein n = 1 (but would also apply if n = 2 or 3 with the corresponding change in the reagent), R terms at the 3 and 9 positions have T = 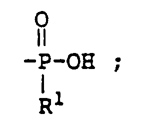 where R¹ = -O-(C₁-C₅ alkyl); and X and Y = H;
the R term at the 6 position has T = 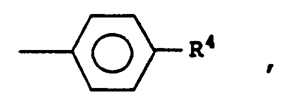 where R⁴ = NO₂ or NH₂; and one of X or Y = H and the other = COOH; and
A, Q and Z = CH.

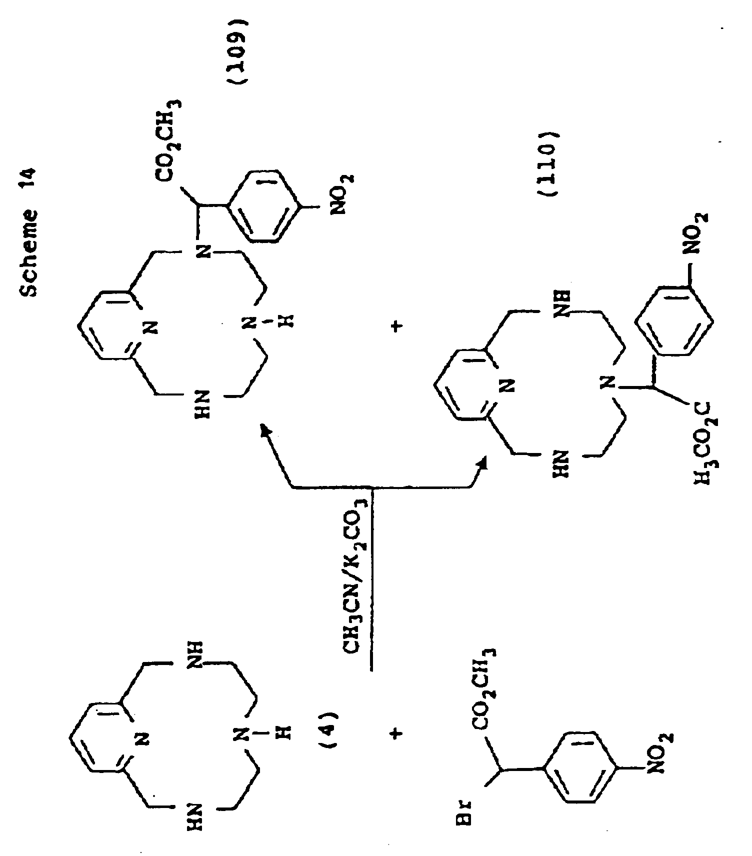

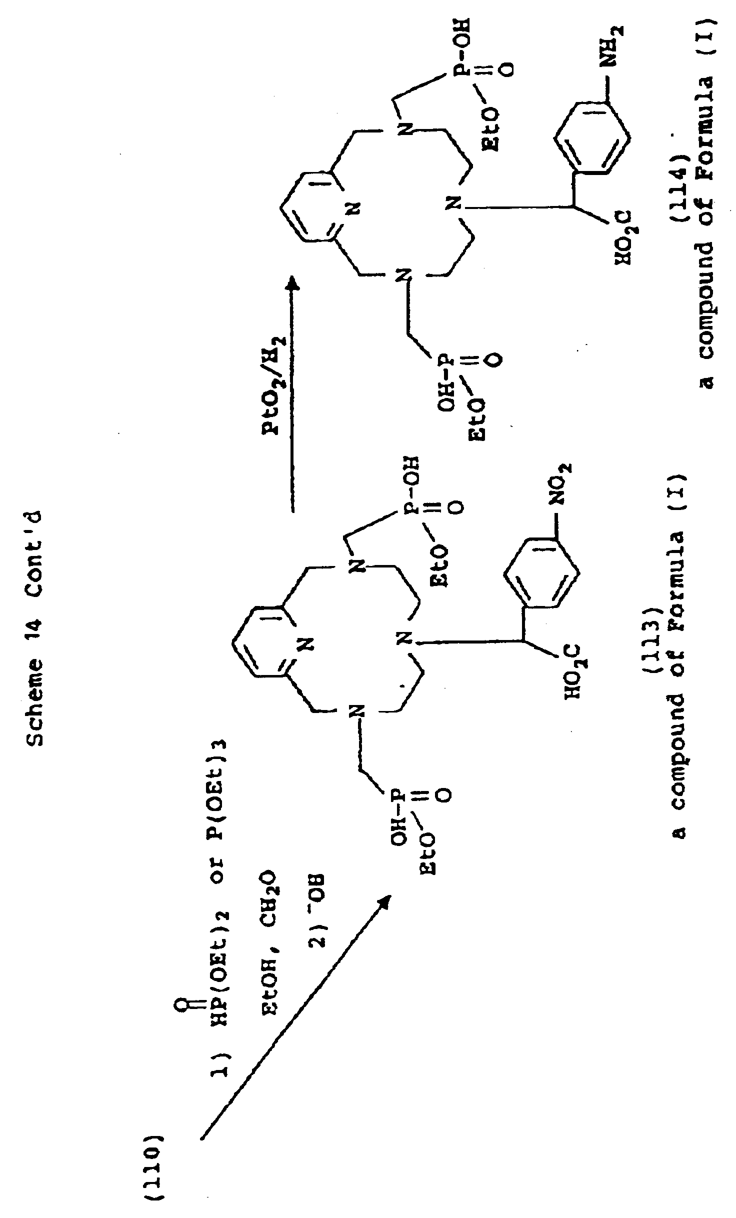

Scheme 15 prepares the compounds of Formula (I) wherein n = 1 (but would also apply if n = 2 or 3 with the corresponding change in the reagent), R terms at the 3 and 6 positions have T = 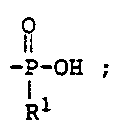 where R¹ = -O-(C₁-C₅ alkyl); and X and Y = H;
the R term at the 9 position has T = 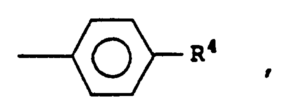 where R⁴ = NO₂ or NH₂; and one of X or Y = H and the other = COOH,
A, Q and Z = CH.

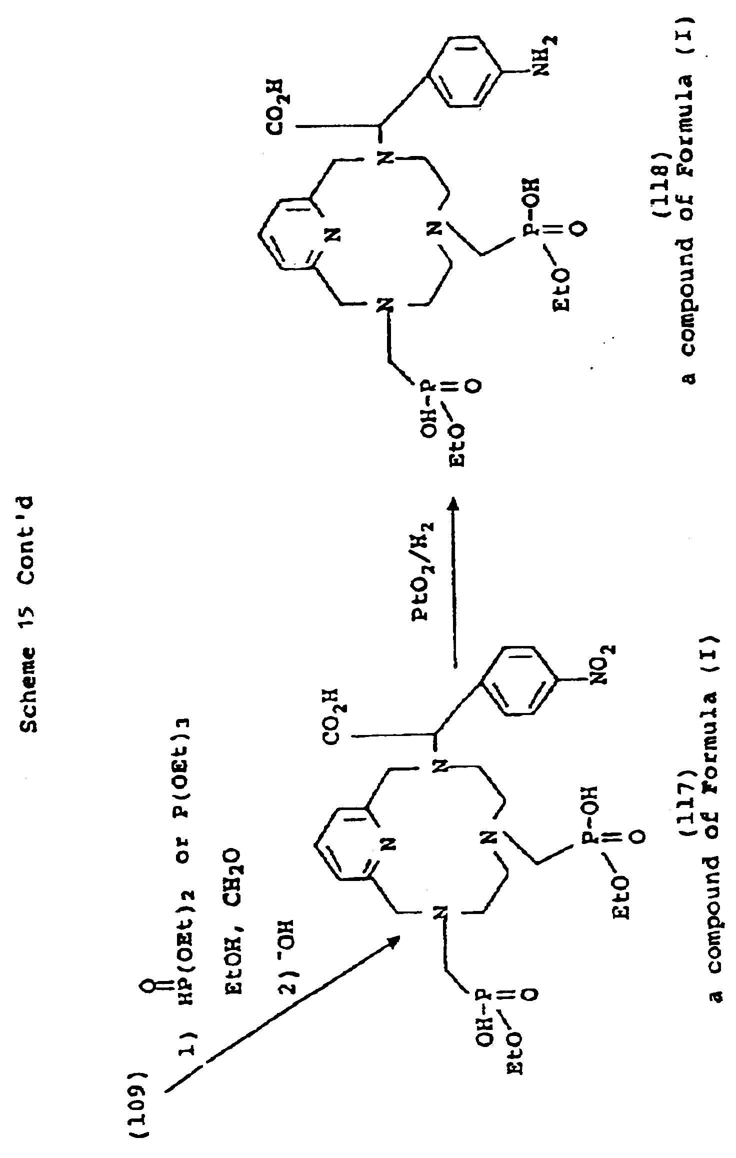

In the above Schemes, the general process description illustrates specific steps that may be used to accomplish a desired reaction step. The general description of these process steps follows.

The synthetic Scheme 1 begins with a halogenation of commercially available bis-pyridyl alcohol (1) using thionyl chloride. Similar procedures for converting an alcohol to an electrophilic substrate, such as treatment with toluenesulfonyl chloride, HBr or HCl, should also result in a similarily reactive product which would work well in subsequent ring closure reactions. Macrocyclization procedures are numerous in the literature and the desired tetraazamacrocycle (3) was prepared according to the method of Stetter et al., Tetrahedron 37, 767-772 (1981). More general procedures have since been published which give good yields of similar macrocycles using milder conditions [A.D. Sherry et al., *J. Org. Chem*. 54, 2990-2992 (1989)]. Detosylation of the intermediate macrocycle [(3) to yield (4)] was accomplished under acidic conditions in good yield. Reductive detosylation procedures are also well known in the literature and can be adapted to the present reaction sequence. Phosphonomethylation to obtain the tris-aminophosphonic acid derivative (5, PCTMP) was conducted under typical Mannich base conditions using phosphorous acid and formaldehyde.

In addition to phosphonic acid derivatives, phosphonate esters [e.g. of formula (6)] can also be prepared under organic conditions in alcohols or aprotic solvents (e.g. acetonitrile, benzene, toluene, tetrahydrofuran) and using the desired dialkylphosphite as the nucleophilic species (see Scheme 2). Depending upon the reactivity of the amine, these reactions may be conducted at a temperature between -10 to 100°C. In addition, trialkylphosphites can be employed under similar Mannich conditions to give the phosphonate ester via oxidation of phosphorous (III) to phosphorous (V) with simultaneous expulsion of one mole of alcohol (Arbuzov reaction). These reactions can be conducted with or without the presence of a solvent When alcohols are employed as the solvent for either dialkyl or trialkyl phosphite reactions, it is beneficial to use the alcohol from which the corresponding phosphonate ester is derived in order to avoid alternative products arising from transesterification. Esters of this type are also prepared via N-alkylation of 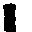 -halodialkylphosphonates in solvents such as acetonitrile, chloroform, dimethylformamide, tetrahydrofuran or 1,4-dioxane with or without the addition of a non-nucleophilic base such as potassium carbonate at room temperature or above. The resulting perester intermediate is then readily hydrolyzed under basic conditions (aqueous hydroxide, pH = 8-14; 30-110°C) to give the corresponding half-acid derivative.

Scheme 3 illustrates an approach to incorporate additional functionality into the pyridine unit of the 12-membered tertaazamacrocycle. Thus, chelidamic acid (Sigma Chemical Company; 12) can be converted to the bis-halomethyl derivative (13) having appropriate substitution at the pyridyl 4-position. Transformations leading to this intermediate are general in nature and its preparation is described by Takalo et al. [*Acta Chemica Scandinavica* B 42, 373-377 (1988)]. Subsequent macrocyclization using this intermediate (15) can be accomplished by the standard DMF reaction at 100°C with the sodiotritosylated triamine, or at room temperature with the tritosylated free base and potassium carbonate, sodium carbonate, or cesium carbonate as base to give products similar to those previously described. Subsequent reactions leading to phosphonate half-acids and phosphinate functionality are identical to those transformations and conditions described in the preceding Schemes.

In Scheme 4, 4-halopyridyl substituted macrocycles (16) are described which can undergo substitution at the 4-position of the pyridyl moiety as described in Scheme 4. Thus, organometallic Pd(II) complexes can be employed to facilitate the coupling reaction between phenylacetylene and phenylacetylene derivatives and the pyridyl macrocycle. Typical reaction conditions for this transformation utilize anhydrous conditions with triethylamine as solvent and at reaction temperature between 10 to 30°C for optimum yields. The identical product can also be obtained using Cu(I) phenylacetylide in anhydrous pyridine at a temperature between 80 to 110°C. In addition, standard anionic alkylation procedures can be employed to affect substitution on the pyridine nucleus with, for example, sodioalkoxides in DMF or dioxane at from 80 to 100°C using bases such as potassium carbonate or sodium hydroxide. Macrocyclic tetraazamacrorycles (24, 25, 26, 27, 28) derivatized in this manner are compatible with transformations described in previous Schemes resulting in analogous phosphonate chelants.

A variation of 4-pyridyl substitution is described in Scheme 5 whereby the 4-hydroxypyridyl moiety (29) is alkylated with a bromoalkylnitrile yielding an intermediate ether linked nitrile (31) which is subsequently incorporated into the macroryclic structure. This type of alkylation procedure is best accomplished under anhydrous conditions in an aprotic solvent such as tetrahydrofuran (THF) and using a non-nucleophilic base such as sodium hydride or butyllithium at temperatures between from -30 to 80°C. The generality of this approach has been described by Chaubet et al., for acyclic analogs [*Tetrahedron Letters* 31(40), 5729-5732 (1990)]. The macrocyclic nitrile prepared in this manner can be reduced to the primary amine (36) by standard procedures followed by protection of the primary amine with 2-(t-butoxycarbonyloxyimino)-2-phenylacetonitrile (BOC-ON; 37). Subsequent functionalization of the macrocyclic secondary amines (38, 39, 40, 41, 42, 43) can then be accomplished by the procedures discussed with the additional requirement that the BOC protecting group be removed using trifluoroacetic acid as described in Scheme 5.

Functionalization can also be carried out on the 3-position of the pyridine ring within the macrocyclic structure as illustrated in Scheme 6. Newkome et al. [*Tetrahedron* 39(12), 2001-2008 (1983)] has previously described the synthesis of ethyl 2,6-halomethylnicotinate (45) which serves as the initial starting material in this synthetic route. Thus, the tris-tosylated macrocycle intermediate (46) can be detosylated under acidic conditions (HBr/AcOH, 25-115°C) with simultaneous hydrolysis to yield the nicotinic acid derivative (48), or reduction of the ester in refluxing ethanol prior to detosylation will result in the 3-hydroxymethyl intermediate (47). The nicotinic acid macrocycle can then be substituted into the general scheme for secondary amine functionalization to yield the various types of phosphonate chelants of Formula (I) (49, 50, 51, 52, 53).

In contrast, the 3-hydroxymethyl analog is advantageously protected prior to functionalization of the macrocyclic amines. The benzyl (Bz) protecting group is shown in Scheme 7 since it must be resistant to the severe acid conditions encountered in the detosylation step. After appropriate functionalization of the secondary amines has been accomplished as described in previous Schemes, the benzyl group is removed under mild catalytic hydrogenation conditions (58).

Macrocyclic derivatives can also be prepared as in Schemes 11-13 where both carboxylate and phosphonate cheating functionalities are present in the same molecule. Thus, varying degrees of carboxylate functionality can be introduced under typical aqueous alkylation procedures using bromoacetic acid. Following this step, the remaining amines can be phosphonomethylated by procedures discussed in previous Schemes using formaldehyde and phosphorous acid, dialkyl phosphonates or trialkyl phosphites.

Schemes 14 and 15 delineate a synthetic approach which introduces an aromatic nitrobenzyl substituent at one of the macrocyclic nitrogen positions. Typically, the macrocyclic amine is mono-N-functionalized in an organic solvent such as acetonitrile or DMF at room temperature using a non-nucleophilic base such as potassium carbonate. Additional functionalization of the remaining nitrogen positions is then performed by methods and conditions described in previous Schemes. After the introduction of the desired chelating moieties, the nitro group is reduced using platinum oxide and hydrogen in water. In this form, the chelating agent is compatible with conjugation techniques which will enable attachment to larger synthetic or natural molecules.

The metal ions used to form the complexes of this invention are Gd⁺³, Mn⁺², Fe⁺³ and available commercially, e.g. from Aldrich Chemical Company. The anion present is halide, preferably chloride, or salt free (metal oxide).

A "paramagnetic nuclide" of this invention means a metal ion which displays spin angular momentum and/or orbital angular momentum. The two types of momentum combine to give the observed paramagnetic moment in a manner that depends largely on the atoms bearing the unpaired electron and, to a lesser extent, upon the environment of such atoms. The paramagnetic nuclides found to be useful in the practice of the invention are gadolinium (Gd⁺³), iron (Fe⁺³) and manganese (Mn⁺²), with Gd⁺³ being preferred.

The complexes are prepared by methods well known in the art. Thus, for example, see Cheating Agents and Metal Chelates, Dwyer & Mellor, Academic Press (1964), Chapter 7. See also methods for making amino acids in Synthetic Production and Utilization of Amino Acids, (edited by Kameko, et al.) John Wiley & Sons (1974). An example of the preparation of a complex involves reacting a bicyclopolyazamacrocyclophosphonic acid with the metal ion under aqueous conditions at a pH from 5 to 7. The complex formed is by a chemical bond and results in a stable paramagnetic nuclide composition, e.g. stable to the disassociation of the paramagnetic nuclide from the ligand.

The complexes of the present invention are administered at a ligand to metal molar ratio of at least 1:1, preferably from 1:1 to 3:1, more preferably from 1:1 to 1.5:1. A large excess of ligand is undesirable since uncomplexed ligand may be toxic to the animal or may result in cardiac arrest or hypocalcemic convulsions.

The antibodies or antibody fragments which may be used in the conjugates described herein can be prepared by techniques well known in the art. Highly specific monoclonal antibodies can be produced by hybridization techniques well known in the art, see for example, Kohler and Milstein [*Nature*, 256, 495-497 (1975); and *Eur. J. Immunol*., 6, 511-519 (1976)]. Such antibodies normally have a highly specific reactivity. In the antibody targeted conjugates, antibodies directed against any desired antigen or hapten may be used. Preferably the antibodies which are used in the conjugates are monoclonal antibodies, or fragments thereof having high specificity for a desired epitope(s). Antibodies used in the present invention may be directed against, for example, tumors, bacteria, fungi, viruses, parasites, mycoplasma, differentiation and other cell membrane antigens, pathogen surface antigens, toxins, enzymes, allergens, drugs and any biologically active molecules. Some examples of antibodies or antibody fragments are 1116-NS-19-9, 1116-NS-3d, 703D4, 704A1, CC49, CC83 and B72.3. All of these antibodies have been deposited in ATCC. A more complete list of antigens can be found in US-A-4,193,983. The conjugates of the present invention are particularly preferred for the diagnosis of various cancers.

This invention is used with a physiologically acceptable carrier, excipient or vehicle therefore. The methods for preparing such formulations are well known. The formulations may be in the form of a suspension, injectable solution or other suitable formulations. Physiologically acceptable suspending media, with or without adjuvants, may be used.

An "effective amount" of the formulation is used for diagnosis. The dose will vary depending on the disease and physical parameters of the animal, such as weight. *In vivo* diagnostics are also contemplated using formulations of this invention.

Other uses of some of the chelants of the present invention may include the removal of undesirable metals (i.e. iron) from the body, attachment to polymeric supports for various purposes, e.g. as diagnostic agents, and removal of metal ions by selective extraction. The ligands of Formula (I) having in at least two R terms T equal to P(O)R¹OH may be used for metal ion control as scale inhibitors. Some of these ligands can be used in less than stoichiometric amounts. Similar uses are known for compounds described in US-A-2,609,390; US-A-3,331,773; US-A-3,336,221; and US-A-3,434,969.

## Claims

1. Bicyclopolyazamacrocyclophosphonic acid compounds of the formula 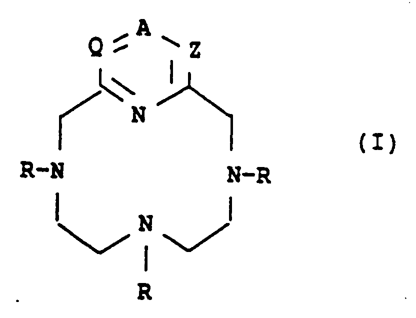 wherein: 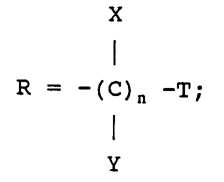 where:
X and Y are independently H, OH, C₁-C₃ alkyl or COOH;
n is an integer of 1, 2 or 3;
with the proviso that: when n is 2, then the sum of X and Y must equal two or more H; and when n is 3, then the sum of X and Y must equal three or more H;
T is H, C₁-C₁₈ alkyl, COOH, OH, SO₃H, 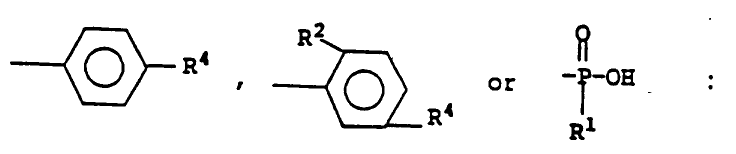
where:
R¹ is -O-(C₁-C₅ alkyl);
R² is H or OH; with the proviso that when R² is OH, then the R term containing the R² must have all X and Y equal to H;
R⁴ is H, NO₂, NH₂, isothiocyanato, semicarbazido, thiosemicarbazido, maleimido, bromoacetamido or carboxyl;
with the proviso that at least one T must be P(O)R¹OH and with the proviso that when one T is 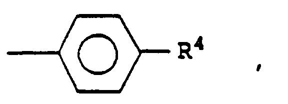 then one X or Y of that R term may be COOH and all other X and Y terms of that R term must be H;
A is CH, N, C-Br, C-Cl, C-OR³, C-OR⁸, N⁺-R⁵X⁻, 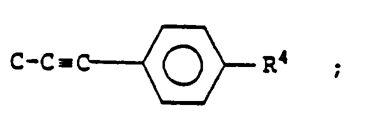
R³ is H, C₁-C₅ alkyl, benzyl, or benzyl substituted with at least one R⁴;
R⁵ is C₁-C₁₆ alkyl, benzyl, or benzyl substituted with at least one R⁴;
R⁸ is C₁-C₁₆ alkylamino;
X⁻ is Cl⁻, Br⁻, I⁻ or H₃CCO₂⁻;
Q and Z independently are CH, N, N⁺-R⁵X⁻, C-CH₂-OR³ or C-C(O)-R⁶,
R⁶ is -O-(C₁-C₃ alkyl), OH or NHR⁷;
R⁷ is C₁-C₅ alkyl or a dextran, a peptide, or a molecule with a specific affinity for a receptor; or pharmaceutically-acceptable salts thereof;
with the proviso that:
a) when Q, A or Z is N or N⁺ -R⁵X⁻, then the other two groups must be CH;
b) when A is C-Br, C-Cl, C-OR³ or C-OR⁸, then both Q and Z must be CH;
c) the sum of the R⁴, R⁷ and R⁸ terms, when present, may not exceed one; and
d) only one of Q or Z can be C-C(O)-R⁶ and when one of Q or Z is C-C(O)-R⁶, then A must be CH.

2. A compound of Claim 1 wherein X and Y are H.

3. A compound of Claim 1 or Claim 2 wherein n is 1.

4. A compound of any one of Claims 1 to 3 wherein A, Q and Z are CH.

5. A compound of any one of the preceding claims wherein in the three R terms T is P(O)R¹OH, where R¹, is as defined in Claim 1.

6. A compound of Claim 5 wherein n is 1

7. A compound of any one of Claims 1 to 4 wherein Q, A and Z are CH; and in the three R terms X, Y and n are defined as in Claim 1, and one T term is 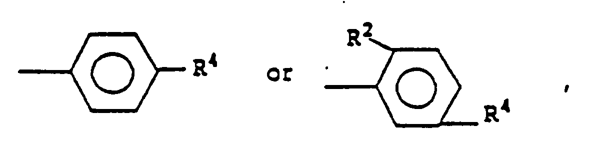 where R² and R⁴ are defined as in Claim 1, and the other two T terms are defined as in Claim 1.

8. A compound of Claim 7 wherein n is 1 and the R term that contains a T moiety which has the R⁴ group present, also has one of X or Y of that R term equal to COOH.

9. A compound of Claim 8 wherein in the two R terms not containing an R⁴ term, all remaining X and Y terms are H.

10. A compound of any one of Claims 1 to 4 wherein n is 1, X and Y are H; T is COOH 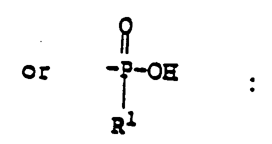 where: R¹ is -O-(C₁-C₅ alkyl).

11. A compound of Claim 10 wherein
(a) Q and Z are CH, A is C-OR³, C-OR⁸, where R³ and R⁸ are defined as in Claim 1, or 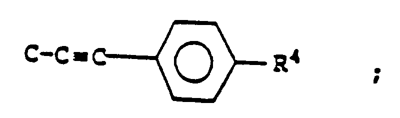 where R⁴ is defined as in Claim 1 or
(b) A is CH, and one of Q or Z is CH and the other is C-C(O)-R⁶, where R⁶ is defined as in Claim 1.

12. A compound of type (b) Claim 11 wherein R⁶ is NHR⁷, where R⁷ is a dextran, a peptide, or a molecule with a specific affinity for a receptor.

13. A compound of Claim 4 wherein one of A, Q or Z is N⁺-R⁵X⁻, where R⁵ and X⁻ are defined as in Claim 1; and in all three terms, the T moiety is P(O)R¹OH, where R¹ is -O-(C₁-C₅ alkyl) and all X and Y terms are H.

14. A complex which comprises a bicyclopolyazamacrocyclophosphonic acid compound as claimed in any one of Claims 1 to 13 complexed with a metal ion selected from Gd⁺³, Mn⁺² or Fe⁺³.

15. A complex of Claim 14 wherein the metal is Gd⁺³.

16. A complex of Claim 14 having an overall negative charge.

17. A complex of Claim 14 having an overall neutral charge.

18. A complex of Claim 14 having an overall charge of +1.

19. A conjugate comprising a bicyclopolyazamacrocyclophosphonic acid complex as claimed in Claim 14 or Claim 15, with the proviso that one of R⁴, R⁷ or R⁸ must be present, and covalently attached to a dextran, a polypeptide, or a molecule that has specific affinity for a receptor.

20. A conjugate as claimed in claim 19, wherein the molecule with a specific affinity for a receptor is an antibody or antibody fragment,

21. A conjugate of Claim 20 wherein the antibody or antibody fragment is a monoclonal antibody or fragment thereof.

22. A conjugate of any one of Claims 19 to 21 wherein A is CH, and one of Q or Z is CH and the other is C-C(O)-R⁶, where R⁶ is NHR⁷, where R⁷ is a biologically active material.

23. A pharmaceutical formulation comprising a complex of any one of Claims 14 to 18 with a pharmaceutically-acceptable carrier.

24. A pharmaceutical formulation comprising a conjugate of any one of Claims 19 to 22 with a pharmaceutically-acceptable carrier.

25. The complex as claimed in any one of Claims 14 to 18 for use as a pharmaceutical.

26. The conjugate as claimed in any one of Claims 19 to 22 for use as a pharmaceutical.

27. A kit for use a a diagnostic agent having as an ingredient a compound as claimed in any one of Claims 1 to 16.

28. A process for preparing a complex as claimed in Claim 14 which comprises reacting a bicyclopolyazamacrocyclophosphonic acid compound as claimed in Claim 1 with a metal ion selected from Gd⁺³, Mn⁺² or Fe⁺³ under aqueous conditions at a pH from 5 to 7.

29. A process for preparing a bicyclopolyazamacrocyclophosphonic acid compound as claimed in Claim 1 which comprises reacting:
(A) a compound of the Formula (I) wherein at least 1 R group is H, with a phosphonating agent; or
(B) a compound of Formula (I) wherein Q, A or Z has a protecting group present, after step (A), removing the blocking group by catalytic hydrogenation or acid.

30. A process of Claim 29 wherein the phosphonating agent has the formula P(OR)₃ where R is defined as in Claim 1.

31. A process of Claim 29 wherein the phosphonating agent has the formula P(OR)₃ where R is defined as in Claim 1, and formaldehyde is a solvent.

## Patentansprüche

1. Bicyclopolyazamacrocyclophosphonsäure-Verbindungen der Formel 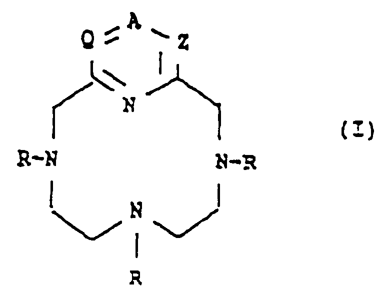 worin 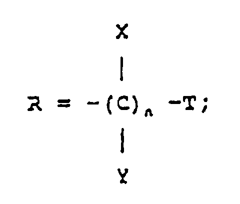 worin
X und Y unabhängig H, OH, C₁-C₃-Alkyl oder COOH sind,
n eine ganze Zahl von 1, 2 oder 3 ist, mit der Maßgabe, daß: wenn n gleich 2 ist, dann die Summe aus X und
Y gleich 2 oder mehr H sein muss, und wenn n gleich 3 ist, dann die Summe aus X und Y gleich 3 oder mehr H sein muss,
T H, C₁-C₁₈-Alkyl, COOH, OH, SO₃H, 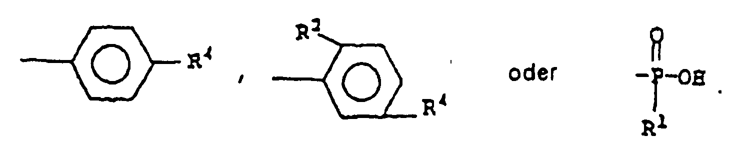
ist, worin:
R¹ -O-[C₁-C₅-Alkyl) ist,
R² H oder OH ist, mit der Maßgabe, daß, wenn R² OH ist, dann der Term R, der das R² enthält, alle X und Y gleich H haben muß,
R⁴ H, NO₂, NH₂, Isothiocyanato, Semicarbazido, Thiosemicarbazido, Maleimido, Bromacetamido oder Carboxyl ist,
mit der Maßgabe, daß mindestens ein T P(O)R¹OH sein muß und mit der Maßgabe, daß, wenn ein T 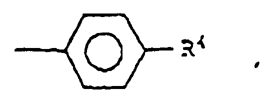 ist, dann ein X oder Y dieses Terms R COOH sein kann und alle anderen
X und Y Terme dieses Terms R H sein müssen,
A CH, N, C-Br, C-Cl, C-OR³, C-OR⁸, N⁺-R⁵X⁻, 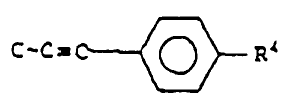 ist,
R³ H, C₁-C₅-Alkyl, Benzyl oder Benzyl substituiert mit mindestens einem R⁴ ist,
R⁵ C₁-C₁₆-Alkyl, Benzyl oder Benzyl substituiert mit mindestens einem R⁴ ist,
R⁸ C₁-C₁₆-Alkylamino ist,
X⁻ Cl⁻, Br⁻, I⁻ oder H₃CCO₂⁻ ist.
Q und Z unabhängig CH, N, N⁺-R⁵X⁻, C-CH₂-OR³ oder C-C(O)-R⁶ sind,
R⁶ -O-(C₁-C₃-Alkyl), OH oder NHR⁷ ist,
R⁷ C₁-C₅-Alkyl oder ein Dextran, ein Peptid oder ein Molekül mit einer spezifischen Affinität für einen Rezeptor ist, oder
pharmazeutisch annehmbare Salze davon,
mit der Maßgabe, daß:
a) wenn Q, A oder Z H oder N⁺-R⁶X⁻ ist, dann die anderen zwei Gruppen CH sein müssen,
b) wenn A C-Br, C-Cl, C-OR³ oder C-OR⁸ ist, dann sowohl Q als auch Z CH sein müssen.
c) die Summe der Terme R⁴, R⁷ und R⁸, wenn vorhanden, nicht größer als 1 sein soll, und
d) nur eines von Q oder Z C-C(O)-R⁶ sein kann und wenn eines von Q oder Z C-C(O)-R⁶ ist, dann A CH sein muss.

2. Verbindung nach Anspruch 1, worin X und Y H sind.

3. Verbindung nach Anspruch 1 oder 2, worin n gleich 1 ist.

4. Verbindung nach einem der Ansprüche 1 bis 3, worin A, Q und Z CH sind.

5. Verbindung nach einem der vorhergehenden Ansprüche, worin in den drei Termen R T P(O)R¹OH ist, worin R¹ wie in Anspruch 1 bestimmt ist.

6. Verbindung nach Anspruch 5, worin n gleich 1 ist.

7. Verbindung nach einem der Ansprüche 1 bis 4, worin Q, A und Z CH sind und in den drei Termen R X, Y und n wie in Anspruch 1 definiert sind und ein Term T ist: 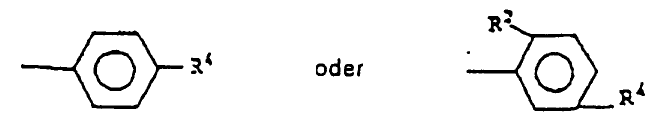 worin R² und R⁴ wie in Anspruch 1 definiert sind und die anderen zwei Terme T wie in Anspruch 1 definiert sind.

8. Verbindung nach Anspruch 7, worin n gleich 1 ist und der Term R, der eine T-Gruppe enthält, in welcher die R⁴-Gruppe vorhanden ist, auch eines der X oder Y dieses Terms R gleich COOH enthält.

9. Verbindung nach Anspruch 8, worin in den zwei Termen R, die keinen Term R⁴ enthalten, alle übrigen X- und Y-Terme H sind.

10. Verbindung nach einem der Ansprüche 1 bis 4, worin n gleich 1 ist, X und Y H sind, T COOH oder 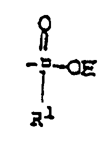 ist, worin R¹ -O-(C₁-C₅-Alkyl) ist.

11. Verbindung nach Anspruch 10, worin
(a) Q und Z CH sind, A C-OR³, C-OR⁸ ist, worin R³ und R⁸ wie in Anspruch 1 definiert sind oder 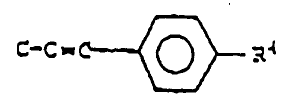 ist,
worin R⁴ wie in Anspruch 1 definiert ist oder
(b) A CH ist und eines von Q oder Z CH ist und das andere C-C(O)-R⁶ ist, worin R⁶ wie in Anspruch 1 definiert ist.

12. Verbindung gemäß Typ (b) des Anspruchs 11, worin R⁶ NHR⁷ ist, worin R⁷ ein Dextran, ein Peptid oder ein Molekül mit einer spezifischen Affinität für einen Rezeptor ist.

13. Verbindung nach Anspruch 4, worin eines von A, Q oder Z N⁺-R⁵X⁻ ist, worin R⁵ und X⁻ wie in Anspruch 1 definiert sind und in allen drei Termen R die T-Gruppe P(O)R¹OH ist, worin R¹ -O-(C₁-C₅-Alkyl) ist und alle X- und Y-Terme H sind.

14. Komplex, der eine Bicyclopolyazamacrocyclophosphonsäure-Verbindung nach einem der Ansprüche 1 bis 13 im Komplex mit einem Metallion, ausgewählt aus Gd⁺³, Mn⁺² oder Fe⁺³ umfaßt.

15. Komplex nach Anspruch 14, worin das Metall Gd⁺³ ist.

16. Komplex nach Anspruch 14 mit einer negativen Gesamtladung.

17. Komplex nach Anspruch 14 mit einer neutralen Gesamtladung.

18. Komplex nach Anspruch 14 mit eIner Gesamtladung von + 1.

19. Konjugat, umfassend einen Bicyclopolyazamacrocyclophosphonsäure-Komplex nach Anspruch 14 oder Anspruch 15, mit der Maßgabe, daß eines von R⁴, R⁷ oder R⁸ vorhanden sein muß und kovalent verbunden ist mit einem Dextran, einem Polypeptid oder einem Molekül, das eine spezifische Affinität für einen Rezeptor hat.

20. Konjugat nach Anspruch 19, worin das Molekül mit einer spezifischen Affinität für einen Rezeptor, einen Antikörper oder ein Antikörperfragment ist.

21. Konjugat nach Anspruch 20, worin der Antikörper oder das Antikörperfragment ein monoklonaler Antikörper oder ein Fragment davon ist.

22. Konjugat nach einem der Ansprüche 19 bis 21, worin A CH ist und eines von Q oder Z CH ist, und das andere C-C(O)-R⁶ ist, worin R⁶ NHR⁷ ist, worin R⁷ ein biologisch aktives Material ist.

23. Pharmazeutische Formulierung, umfassend einen Komplex nach einem der Ansprüche 14 bis 18 mit einem pharmazeutisch verträglichen Träger.

24. Pharmazeutische Formulierung, umfassend ein Konjugat nach einem der Ansprüche 19 bis 22 mit einem pharmazeutisch verträglichen Träger.

25. Komplex nach einem der Ansprüche 14 bis 18 zur Verwendung als Pharmazeutikum.

26. Konjugat nach einem der Ansprüche 19 bis 22 zur Verwendung als Pharmazeutikum.

27. Kit zur Verwendung als ein diagnostisches Mittel, der als einen Bestandteil eine Verbindung nach einem der Ansprüche 1 bis 13 aufweist.

28. Verfahren zur Herstellung eines Komplexes nach Anspruch 14, umfassend umsetzen einer Bicyclopolyazamacrocyclophosphonsäure-Verbindung nach Anspruch 1 mit einem Metallion ausgewählt aus Gd⁺³, Mn⁺² oder Fe⁺³ unter wäßrigen Bedingungen bei einem pH von 5 bis 7.

29. Verfahren zur Herstellung einer Bicyclopolyazamacrocyclophosphonsäure-Verbindung nach Anspruch 1, das umfasst Umsetzen:
(A) einer Verbindung der Formel (I), worin mindestens eine R-Gruppe H ist, mit einem phosphonierenden Mittel, oder
(B) einer Verbindung der Formel (I), worin Q, A oder Z nach Schritt (A) eine Schutzgruppe enthält, wobei die Blockierungsgruppe durch katalytische Hydrogenierung oder Säure entfernt wird.

30. Verfahren nach Anspruch 29, worin das phosphonierende Mittel die Formel P(OR)₃ hat, worin R wie in Anspruch 1 definiert ist.

31. Verfahren nach Anspruch 29, worin das phosphonierende Mittel die Formel P(OR)₃ hat, worin R wie in Anspruch 1 definiert ist und Formaldehyd ein Lösungsmittel ist.

## Revendications

1. Composés de type acides bicyclopolyazamacrocyclophosphoniques répondant à la formule : 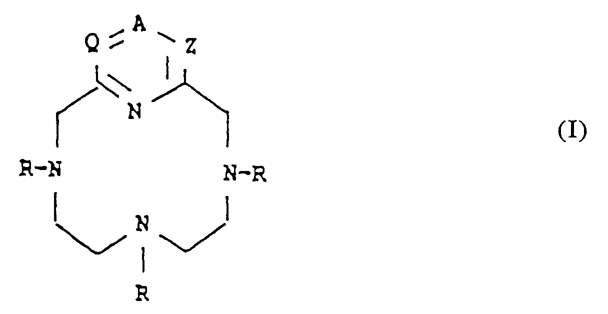 dans laquelle 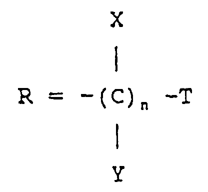
où X et Y représentent indépendamment H, OH, un groupe alkyle en C₁-C₃ ou COOH;
n est un nombre entier de 1, 2 ou 3 ;
à condition que, quand n vaut 2, alors l'ensemble des X et Y représente deux H ou plus ; et quand n vaut 3, l'ensemble des X et Y représente trois H ou plus ;
T représente H, un groupe alkyle en C₁-C₃, COOH, OH, SO₃H, 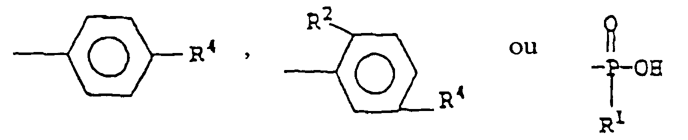
dans lesquelles R¹ représente -O-(alkyle en C₁-C₅) ;
R² représente H ou OH, à condition que, quand R² représente OH dans le radical R contenant R², alors tous les X et Y représentent H ;
R⁴ représente H, NO₂, NH₂, les groupes isothiocyanato, semicarbazido, thiosemicarbazido, maléimido, bromoacétamido ou carboxyle ;
à condition qu'au moins un T représente P(O)R¹OH et à condition que, quand un symbole T représente : 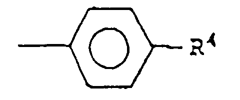
alors un symbole X ou Y contenu dans le radical R peut représenter COOH et tous les autres symboles X et Y dans le radical R doivent représenter H ;
A représente CH, N, C-Br, C-Cl, C-OR³, C-OR⁸, N⁺-R⁵X⁻, 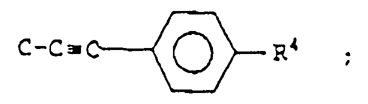
R³ représente H, un groupe alkyle en C₁-C₅, un groupe benzyle ou benzyle substitué par au moins un R⁴ ;
R⁵ représente un groupe alkyle en C₁-C₁₆, benzyle ou benzyle substitué par au moins un R⁴ ;
R⁸ représente un groupe alkylamino en C₁-C₁₆ ;
X⁻ représente Cl⁻, Br⁻, I⁻ ou H₃CCO₂⁻ ;
Q et Z représentent indépendamment CH, N, N⁺-R⁵X⁻, C-CH₂-OR³ ou C-C(O)-R⁶;
R⁶ représente -O-(alkyle en C₁-C₃), OH ou NHR⁷ ;
R⁷ représente un groupe alkyle en C₁-C₅ ou un dextrane, un peptide ou une molécule présentant une affinité spécifique pour un récepteur ; ou sels pharmaceutiquement acceptables de ces derniers ;
à condition que :
a) quand Q, A ou Z représente N ou N⁺-R⁵X⁻, alors les deux autres groupes représentent CH ;
b) quand A représente C-Br, C-Cl, C-OR³ ou C-OR⁸, alors Q et Z représentent CH ;
c) la somme des symboles R⁴, R⁷ et R⁸, s'ils sont présents, ne dépasse pas un ; et
d) seulement l'un des Q et Z représente C-C(O)-R⁶ et quand l'un des Q et Z représente C-C(O)-R⁶, alors A représente CH.

2. Composé conforme à la revendication 1, dans lequel X et Y représentent H.

3. Composé conforme à la revendication 1 ou 2, dans lequel n vaut 1.

4. Composé conforme à l'une quelconque des revendications 1 à 3, dans lequel A, Q et Z représentent CH.

5. Composé conforme à l'une quelconque des précédentes revendications, dans lequel les symboles T dans les trois radicaux R représentent P(O)R¹OH où R¹ est tel que défini dans la revendication 1.

6. Composé conforme à la revendication 5, dans lequel n vaut 1.

7. Composé conforme à l'une quelconque des revendications 1 à 4, dans lequel Q, A et Z représentent CH, et les symboles X, Y et n dans les trois radicaux R sont tels que définis dans la revendication 1, et un symbole T représente 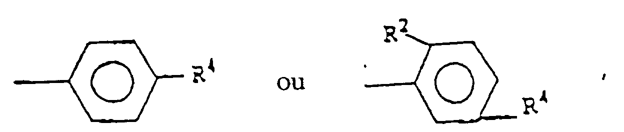 dans lesquels R² et R⁴ sont tels que définis dans la revendication 1, et les deux autres symboles T sont tels que définis dans la revendication 1.

8. Composé conforme à la revendication 7, dans lequel n vaut 1 et le radical R qui contient un fragment T comportant le groupe R⁴, comporte aussi l'un des X et Y représentant COOH.

9. Composé conforme à la revendication 8, dans lequel dans les deux radicaux R ne contenant pas un groupe R⁴, tous les X et Y restant représentent H.

10. Composé conforme à l'une quelconque des revendications 1 à 4, dans lequel n vaut 1, X et Y représentent H, T représente COOH ou 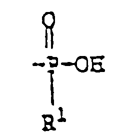 où R¹ représente -O-(alkyle en C₁-C₅).

11. Composé conforme à la revendication 10, dans lequel
(a) Q et Z représentent CH, A représente C-OR³, C-OR⁸, où R³ et R⁸ sont tels que définis dans la revendication 1, ou 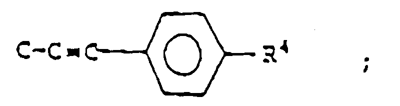 où R⁴ est tel que défini dans la revendication 1, ou
(b) A représente CH, et l'un des Q ou Z représente CH, et l'autre symbole représente C-C(O)-R⁶, où R⁶ est tel que défini dans la revendication 1.

12. Composé de type (b) de la revendication 11, dans lequel R⁶ représente NHR⁷, où R⁷ représente un dextrane, un peptide ou une molécule présentant une affinité spécifique pour un récepteur.

13. Composé conforme à la revendication 4, dans lequel l'un des A, Q et Z représente N⁺-R⁵X⁻ où R⁵ et X⁻ sont tels que définis dans la revendication 1, et le fragment T dans les trois radicaux R représente P(O)R¹OH, où R¹ représente -O-(alkyle en C₁-C₅), et tous les symboles X et Y représentent H.

14. Complexe qui comprend un composé de type acide bicyclopolyazamacrocyclophosphonique conforme à l'une quelconque des revendications 1 à 13, complexé avec un ion métallique choisi parmi Gd³⁺, Mn²⁺ ou Fe³⁺.

15. Complexe conforme à la revendication 14, dans lequel l'ion métallique est Gd³⁺.

16. Complexe conforme à la revendication 14, présentant une charge totale négative.

17. Complexe conforme à la revendication 14, présentant une charge totale neutre.

18. Complexe conforme à la revendication 14, présentant une charge totale +1.

19. Composé conjugué comprenant un complexe d'acide bicyclopolyazamacrocyclophosphonique conforme à la revendication 14 ou 15, à condition que l'un des R⁴, R⁷ ou R⁸ puisse être présent et lié de manière covalente à un dextrane, un polypeptide ou à une molécule présentant une affinité spécifique pour un récepteur.

20. Composé conjugué conforme à la revendication 19, dans lequel la molécule présentant une affinité spécifique pour un récepteur est un anticorps ou un fragment d'anticorps.

21. Composé conjugué conforme à la revendication 20, dans lequel l'anticorps ou le fragment d'anticorps est un anticorps monoclonal ou un fragment de ce dernier.

22. Composé conjugué conforme à l'une quelconque des revendications 19 à 21, dans lequel A représente CH et l'un des Q et Z représente CH, et l'autre représente C-C(O)-R⁶ où R⁶ représente NHR⁷ où R⁷ est un matériau biologiquement actif.

23. Formulation pharmaceutique comprenant un complexe conforme à l'une quelconque des revendications 14 à 18, et un véhicule pharmaceutiquement acceptable.

24. Formulation pharmaceutique comprenant un composé conjugué conforme à l'une quelconque des revendications 19 à 22, et un véhicule pharmaceutiquement acceptable.

25. Complexe conforme à l'une quelconque des revendications 14 à 18, destiné à être utilisé comme produit pharmaceutique.

26. Composé conjugué conforme à l'une quelconque des revendications 19 à 22, destiné à être utilisé comme produit pharmaceutique.

27. Trousse destinée à être utilisée comme agent diagnostique ayant comme ingrédient un composé conforme à l'une quelconque des revendications 1 à 13.

28. Procédé pour préparer un complexe conforme à la revendication 14, qui comprend la réaction d'un composé de type acide bicyclopolyazamacrocyclophosphonique conforme à la revendication 1, avec un ion métallique choisi parmi Gd³⁺, Mn²⁺ et Fe³⁺, dans un milieu aqueux à un pH de 5 à 7.

29. Procédé pour préparer un composé de type acide bicyclopolyazamacrocyclophosphonique conforme à la revendication 1, qui comprend la réaction :
(A) d'un composé répondant à la formule (I) dans laquelle au moins un groupe R représente H, avec un agent de phosphonatation ; ou
(B) d'un composé répondant à la formule (I) dans laquelle Q, A ou Z comporte un groupe de protection, après l'étape (A), l'élimination du groupe bloquant par un acide ou hydrogénation catalytique.

30. Procédé conforme à la revendication 29, dans lequel l'agent de phosphonatation répond à la formule P(OR)₃ où R est tel que défini dans la revendication 1.

31. Procédé conforme à la revendication 29, dans lequel l'agent de phosphonatation répond à la formule P(OR)₃ où R est tel que défini dans la revendication 1, et le formaldéhyde est un solvant.
